# EUROPEAN PATENT APPLICATION

(11) **EP 1 961 450 A1**
(43) Date of publication of application: **27.08.2008**
(21) Application number: 07102728.8
(22) Date of filing: 20.02.2007
(51) Int. Cl.: A61Q 5/06, A61Q 5/10, A61Q 5/08, A61K 8/81

(54) **Dyes for keratinic fibers comprising a special anionic thickener**

(71) Applicant: Wella Aktiengesellschaft, 64274 Darmstadt (DE)
(72) Inventor: Schmenger, Jürgen, 64331 Weiterstadt (DE); Kujawa, Jolanthe, 64289 Darmstadt (DE); Bonn, Melanie, 64285 Darmstadt (DE)
(74) Representative: Bockhorst, Matthias

(57) **Abstract**

The invention relates to compositons for the oxidative or non-oxidative dyeing of keratin fibers, compositions for the simultaneously lightening and coloring of keratin fibers or compositions for the bleaching of keratin fibers, comprising an anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer), as well as the use of the above anionic acrylic thickener to thicken compositons for the oxidative or non-oxidative dyeing of keratin fibers, compositions for the simultaneously lightening and coloring of keratin fibers or compositions for the bleaching of keratin fibers.

## Description

### FIELD OF THE INVENTION

The subject matter of the present application are compositions and methods for dyeing keratin fibers comprising a special anionic acrylic polymer as thickener.

### BACKGROUND OF THE INVENTION

The object of the invention are thickened compositions for dyeing and/or lighthening/bleaching keratin fibers, particularly human hair, that contain direct and/or oxidation dyes and/or bleaching/oxidizing agents and a special thickener, as well as a method for coloring and/or bleaching hair by use of such compositions,
Coloring and bleaching compositions are usually in the form of aqueous -preferably thickened-solutions or emulsions. Oxidation dyes and bleaching compositions as a rule consist of two components (i) a carrier composition containing the dyes or the bleaching agent and (ii) an oxidizing agent, said components being mixed with one another prior to use and then applied to the hair. Mixing will produce a higher or lower viscosity, depending on the viscosity and mixing ratio of the two components. Normally a good adhesion (i.e. a higher viscosity) of the ready-to-use composition is appreciated. In addition, the hairdressers often needs for their work higher viscosities, for example for special strand or sheet techniques and when special work is to be done with the dye brush or the highlighting brush.

### SUMMARY OF THE INVENTION

Hence, a great need existed for economical thickening of the compositions that would ensure good miscibility of said carrier composition with the oxidant and would afford colorants/bleaching compositions with good adhesion properties and coloring/bleaching characteristics.

### DETAILED DESCRIPTION OF THE INVENTION

It has now been found that the aforesaid problem can be solved by the use of special anionic acrylic polymers as described in WO 01/97772.

The present invention therefore relates to
a) an agent for the oxidative coloring of keratin fibers, in particular human hair, comprising at least one oxidative dye precursor and at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer), preferably from 6 to 35 percent, preferably from 8 to 30 percent;
b) an agent for the non-oxidative coloring of keratin fibers, in particular human hair, comprising at least one direct dye and at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer), preferably from 6 to 35 percent, preferably from 8 to 30 percent;
c) an agent for the simultaneously lightening and coloring of keratin fibers, in particular human hair, comprising at least one bleaching agent (e.g. persulfates), at least one dye which is stable in the presence of the used oxidizing agent, and at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer), preferably from 6 to 35 percent, preferably from 8 to 30 percent;
d) an agent for the bleaching of keratin fibers, in particular human hair, comprising at least one bleaching agent (e.g. persulfates) and at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer), preferably from 6 to 35 percent, preferably from 8 to 30 percent;
e) an agent for the simultaneously lightening and coloring of keratin fibers, in particular human hair, which is mixed with an oxidizing agent (e.g. hydrogen peroxide) prior to use, comprising at least one dye which is stable in the presence of the used oxidizing agent, and at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer), preferably from 6 to 35 percent, preferably from 8 to 30 percent.

Especially preferred are anionic acrylic polymers wherein said monomer performing a weak acid function is selected from the group consisting of acrylic acid, methacrylic acid, itaconic acid, crotonic acid, maleic acid and fumaric acid and said monomer performing a strong acid function is selected from the group consisting of monomers having a function of the sulfonic acid type or phosponic acid type, such as 2-acrylamido-2-methylpropane sulfonic acid (AMPS).

Furthermore the anionic acrylic polymer may be crosslinked (or branched) by one of the following crosslinking agents: methylene bisacrylamide (MBA), ethylene glycol diacrylate, polyethylene glycol dimethacrylate, diacrylamide, cyanomethacrylate, vinyloxyethacrylate or methacrylate, formaldehyde, glyoxal, and compositions of the glycidylether type such as ethyleneglycol diglycidylether, or epoxydes.

Particularly preferred anionic acrylic thickeners are Sodium Acrylate/Sodium Acryloyldimethyl Taurate Copolymers. A articularly suitable anionic acrylic thickeners with the INCI name Sodium Acrylate/Sodium Acryloyldimethyl Taurate Copolymer & Hydrogenated Polydecene & Sorbitan Laurate & Trideceth-6 is marketed by Arch Personal Care Products, South Plainfield, USA under the tradename ViscUp® EZ. For further details of the structure and the preparation of said anionic acrylic thickeners reference is made to WO 01/97772; that herewith is explicitly incorporated herein by reference.

The anionic acrylic thickener is preferably contained in the agent of the invention in an amount from 0.01 to 8.0 percent by weight and particularly from 0.1 to 5 percent by weight (based on the active substance).

If the agent according to the invention is the agent for the oxidative coloring of keratin fibers (a) it comprises oxidative hair dye precursors that will deliver a variety of hair colors to the hair. These small molecules are activated by an oxidizing agent or air oxygen and react with further molecules to form a larger colored complex in the hair shaft. Suitable oxidative hair dye precursors are developers, couplers and compounds coupling with themselves The developers may be used alone or in combination with other developers (also known as primary intermediates), and one or more may be used in combination with one or more couplers.

Couplers (also known as color modifiers or secondary intermediates) are generally colorless molecules that can form colors in the presence of activated developers, and are used with other developers or couplers to generate specific color effects or to stabilize the color. The choice of developers and couplers will be determined by the color, shade and intensity of coloration that is desired. The developers and couplers may be used herein, singly or in combination, to provide dyes having a variety of shades ranging from ash blonde to black.

Suitable oxidative hair dye precursors are, for example, the following developers, couplers and compounds coupling with themselves:
(i) Developer substances: p-phenylenediamine derivatives, e.g. benzene-1,4-diamine (commonly known as p-phenylenediamine), 2-methyl-benzene-1,4-diamine, 2-chloro-benzene-1,4-diamine, N-phenyl-benzene-1,4-diamine, 1,4-diamino-2-methoxymethylbenzene and physiologically compatible salts thereof, N-(2-ethoxyethyl)benzene-1,4-diamine, 2-[(4-amino-phenyl)-(2-hydroxy-ethyl)-amino]-ethanol, (commonly known as N,N-bis(2-hydroxyethyl)-p-phenylenediamine) (2,5-diamino-phenyl)-methanol, 1-(2'-Hydroxyethyl)-2,5-diaminobenzene, 2-(2,5-diamino-phenyl)-ethanol, N-(4-aminophenyl)benzene-1,4-diamine, 2,6-dimethyl-benzene-1,4-diamine, 2-isopropyl-benzene-1,4-diamine, 1-[(4-aminophenyl)amino]-propan-2-ol, 2-propyl-benzene-1,4-diamine, 1,3-bis[(4-aminophenyl)(2-hydroxyethyl)amino]propan-2-ol, N⁴,N⁴,2-trimethylbenzene-1,4-diamine, 2-methoxy-benzene-1,4-diamine, 1-(2,5-diaminophenyl)ethane-1,2-diol, 2,3-dimethyl-benzene-1,4-diamine, N-(4-amino-3-hydroxyphenyl)-acetamide, 2,6-diethylbenzene-1,4-diamine, 2,5-dimethylbenzene-1,4-diamine, 2-thien-2-ylbenzene-1,4-diamine, 2-thien-3-ylbenzene-1,4-diamine, 2-pyridin-3-ylbenzene-1,4-diamine, 1,1'-biphenyl-2,5-diamine, 2-(methoxymethyl)-benzene-1,4-diamine, 2-(aminomethyl)benzene-1,4-diamine, 2-(2,5-diaminophenoxy)ethanol, N-[2-(2,5-diaminophenoxy)ethyl]-acetamide, N,N-dimethylbenzene-1,4-diamine, N,N-diethylbenzene-1,4-diamine, N,N-dipropylbenzene-1,4-diamine, 2-[(4-aminophenyl)-(ethyl)amino]ethanol, 2-[(4-amino-3-methyl-phenyl)-(2-hydroxyethyl)-amino]-ethanol, N-(2-methoxyethyl)-benzene-1,4-diamine, 3-[(4-aminophenyl)amino]propan-1-ol, 3-[(4-aminophenyl)-amino]propane-1,2-diol, N-{4-[(4-aminophenyl)amino]butyl}benzene-1,4-diamine, and 2-[2-(2- {2-[(2,5-diaminophenyl)-oxy]ethoxy}ethoxy)ethoxy]benzene-1,4-diamine; 1,3-Bis(N(2-Hydroxyethyl)-N-(4-aminophenyl)amino)-2-propanol; 2,2'-[1,2-Ethanediyl-bis-(oxy-2,1-ethanediyloxy)]-bis-benzene-1,4-diamine; N,N-Bis(2-hydroxyethyl)-p-phenylinediamine; p-aminophenol derivatives such as: 4-amino-phenol (commonly known as p-aminophenol), 4-methylamino-phenol, 4-amino-3-methyl-phenol, 4-amino-2-hydroxymethyl-phenol, 4-amino-2-methyl-phenol, 4-amino-1-hydroxy-2-(2'-hydroxyethylaminomethyl)benzene, 4-amino-2-methoxymethyl-phenol, 5-amino-2-hydroxy-benzoic acid, 1-(5-amino-2-hydroxy-phenyl)-ethane-1,2-diol, 4-amino-2-(2-hydroxyethyl)-phenol, 4-amino-3-(hydroxymethyl)-phenol, 4-amino-3-fluoro-phenol, 4-amino-2-(aminomethyl)-phenol, 4-amino-2-fluoro-phenol; 1-Hydroxy-2,4-diaminobenzene; 1-(2'-Hydroxyethyloxy)-2,4-diaminobenzene; 2,4-Diamino-5-methylphenetol; o-phenylenediamine derivatives such as: 3,4-Diaminobenzoic acid and salts thereof; o-aminophenol derivatives such as: 2-amino-phenol (commonly known as o-aminophenol), 2,4-diaminophenol, 2-amino-5-methyl-phenol, 2-amino-6-methyl-phenol, N-(4-amino-3-hydroxy-phenyl)-acetamide, and 2-amino-4-methyl-phenol; and heterocyclic derivatives such as: pyrimidine-2,4,5,6-tetramine (commonly known as 2,4,5,6-tetraamino-pyridine), 1-methyl-1H-pyrazole-4,5-diamine, 2-(4,5-diamino-1H-pyrazol-1-yl)ethanol, N²,N²-dimethyl-pyridine-2,5-diamine, 2-[(3-amino-6-methoxypyridin-2-yl)amino]ethanol, 6-methoxy-N²-methyl-pyridine-2,3-diamine, 2,5,6-triaminopyrimidin-4(1H)-one, pyridine-2,5-diamine, 1-isopropyl-1H-pyrazole-4,5-diamine, 1-(4-methylbenzyl)-1H-pyrazole-4,5-diamine, 1-(benzyl)-1H-pyrazole-4,5-diamine, 1-(4-chlorobenzyl)-1H-pyrazole-4,5-diamine, pyrazolo[1,5-a]-pyrimidine-3,7-diamine, 5,6,7-trimethylpyrazolo[1,5-a]pyrimidin-3-ylamine hydrochloride, 7-methylpyrazolo[1,5-a]pyrimidin-3-ylamine hydrochloride, 2,5,6,7-teramethyl-pyrazolo[1,5-a]pyrimidin-3-ylamine hydrochloride, 5,7-di-tert-butylpyrazolo[1,5-a]pyrimidin-3-ylamine hydrochloride, 5,7-ditrifluoromethyl-pyrazolo[1,5-a]pyrimidin-3-ylamine hydrochloride, 2-methylpyrazolo[1,5-a]pyrimidin-3,7-diamine hydrochloride; 4-hydroxy-2,5,6-triaminopyrimidine; 1-(2'hydroxyethyl)-amino-3,4-methylene dioxybenzene; and 1-hydroxyethyl-4,5-diaminopyrazole sulphate.
   Additional developers include: *N*-(3-furylmethyl)benzene-1,4-diamine; N-Thiophen-3-ylmethyl-benzene-1,4-diamine; *N*-(2-furylmethyl)benzene-1,4-diamine; N-Thiophen-2-ylmethylbenzene-1,4-diamine; 4-Hydroxy-benzoic acid (2,5-diamino-benzylidene)-hydrazide; 3-(2,5-Diamino-phenyl)-N-ethyl-acrylamide; 2-[3-(3-Amino-phenylamino)-propenyl]-benzene-1,4-diamine; 2-[3-(4-Amino-phenylamino)-propenyl]-benzene-1,4-diamine; 2-(6-Methyl-pyridin-2-yl)-benzene-1,4-diamine; 2-Pyridin-2-yl-benzene-1,4-diamine; 2-[3-(4-Amino-phenylamino)-propenyl]-benzene-1,4-diamine; 2-[3-(3-Amino-phenylamino)-propenyl]-benzene-1,4-diamine; 3-(2,5-Diamino-phenyl)-N-ethyl-acrylamide; 2-Thiazol-2-yl-benzene-1,4-diamine; 4-Hydroxy-benzoic acid (2,5-diamino-benzylidene)-hydrazide; 3'-Fluoro-biphenyl-2,5-diamine; 2-Propenylbenzene-1,4-diamine; 2'-Chloro-biphenyl-2,5-diamine; N-Thiophen-3-ylmethyl-benzene-1,4-diamine; *N*-(3-furylmethyl)benzene-1,4-diamine; 4'-Methoxy-biphenyl-2,5-diamine; N-(4-Amino-benzyl)-benzene-1,4-diamine; 2-Methyl-5-[(1-H-pyrrol-2-ylmethyl)-amino]-phenol; 5-[(Furan-2-ylmethyl)-amino]-2-methyl-phenol; 5-Isopropylamino-2-methyl-phenol; Biphenyl-2,4,4'-triamine hydrochloride; 5-(4-Amino-phenyl)aminomethyl-benzene-1,3-diamine hydrochloride; 5-Phenylaminomethyl-benzene-1,3-diamine hydrochloride; 2-[4-Amino-2-(3,5-diamino-benzylamino)-phenoxy]-ethanol hydrochloride; 5-(3-Amino-phenyl)aminomethylbenzene-1,3-diamine hydrochloride; N-(2-Amino-benzyl)-benzene-1,3-diamine hydrochloride; N-Furan-2-ylmethyl-benzene-1,3-diamine hydrochloride; 2-[(3-Amino-phenylamino)-methyl]-phenol hydrochloride; 4-Amino-2-propylaminomethyl-phenol; hydrochloride; N-Benzo[1,3]dioxol-5-ylmethyl-benzene-1,3-diamine hydrochloride; N-[4-Amino-2-(2-hydroxyethyl)-2H-pyrazol-3-yl]-3-(5-amino-2-hydroxy-phenyl)-acrylamide; hydrochloride; 4-Amino-2-(isopropylamino-methyl)-phenol; hydrochloride; 4-Thiophen-3-yl-benzene-1,3-diamine; hydrochloride hydrochloride; 5-Phenylaminomethyl-benzene-1,3-diamine hydrochloride; 5-(3-Amino-phenyl)aminomethyl-benzene-1,3-diamine hydrochloride; 4-Thiophen-3-yl-benzene-1,3-diamine; hydrochloride; 2',4'-Diamino-biphenyl-4-ol; hydrochloride; 5-Cyclobutylamino-2-methyl-phenol; 5-Cyclobutylamino-2-methyl-phenol; 4-Amino-2-(pyridin-3-ylaminomethyl)-phenol; 5-(3-Amino-phenyl)aminomethyl-benzene-1,3-diamine hydrochloride; 5-Allylaminomethyl-benzene-1,3-diamine hydrochloride; N-(4-Amino-benzyl)-benzene-1,3-diamine hydrochloride; N-Benzyl-benzene-1,3-diamine hydrochloride; 3-[(3-Amino-phenylamino)-methyl]-phenol hydrochloride; N-(4-Methoxy-benzyl)-benzene-1,3-diamine hydrochloride; N-Thiophen-2-ylmethyl-benzene-1,3-diamine hydrochloride; 4-Amino-2-[(2-hydroxy-5-nitro-phenylamino)-methyl]-phenol; hydrochloride; 2',4'-Diamino-biphenyl-4-ol hydrochloride; Biphenyl-2,4,4'-triamine; 5-(4-Amino-phenyl)aminomethyl-benzene-1,3-diamine hydrochloride; 2-[4-Amino-2-(3,5-diamino-benzylamino)-phenoxy]-ethanol hydrochloride; 5-Allylaminomethyl-benzene-1,3-diamine hydrochloride; 5-(3-Aminophenyl)aminomethyl-benzene-1,3-diamine hydrochloride; N-(4-Amino-benzyl)-benzene-1,3-diamine hydrochloride; N-Benzyl-benzene-1,3-diamine hydrochloride; 3-[(3-Aminophenylamino)-methyl]-phenol hydrochloride; N-(2-Amino-benzyl)-benzene-1,3-diamine hydrochloride; N-(4-Methoxy-benzyl)-benzene-1,3-diamine hydrochloride; N-Furan-2-ylmethylbenzene-1,3-diamine hydrochloride; 2-[(3-Amino-phenylamino)-methyl]-phenol hydrochloride; N-Thiophen-2-ylmethyl-benzene-1,3-diamine hydrochloride; N-Benzo[1,3]dioxol-5-ylmethylbenzene-1,3-diamine hydrochloride; N-[4-Amino-2-(2-hydroxy-ethyl)-2H-pyrazol-3-yl]-3-(5-amino-2-hydroxy-phenyl)-acrylamide hydrochloride; 4-Amino-2-propylaminomethyl-phenol; hydrochloride; 4-Amino-2-(isopropylamino-methyl)-phenol hydrochloride; 4-Amino-2-[(2-hydroxy-5-nitro-phenylamino)-methyl]-phenol hydrochloride; 2-Methyl-5-[(1-H-pyrrol-2-ylmethyl)-amino]-phenol; 5-[(Furan-2-ylmethyl)-amino]-2-methyl-phenol; 5-Isopropylamino-2-methyl-phenol; 5-Cyclobutylamino-2-methyl-phenol; 4-Amino-2-(pyridin-3-ylaminomethyl)-phenol; and 5-Cyclobutylamino-2-methyl-phenol.
   The following compounds are especially suitable as developer compounds: 1,4-diamino-2,6-dimethylbenzene, 1,4-diamino-2,5-dimethylbenzene, 1,4-diamino-2,3-dimethylbenzene, 2-chloro-1,4-diaminobenzene, 4-phenylaminoaniline, 1,4-diamino-2-methoxymethylbenzene, 4-dimethylaminoaniline, 4-diethylamino-aniline, 4-[di(2-hydroxyethyl)amino]aniline, 4-[(2-methoxyethyl)amino]-aniline, 4-[(3-hydroxypropyl)amino]aniline, 1,4-diamino-2-(2-hydroxyethyl)benzene, 1,4-diamino-2-(1-methylethyl)benzene, 1,3-bis-[(4-aminophenyl)-(2-hydroxyethyl)amino]-2-propanol, 1,8-bis-(2,5-diaminophenoxy)-3,6-dioxaoctane, 4-aminophenol, 4-amino-3-methylphenol, 4-methyl-aminophenol, 4-amino-2-(aminomethyl)phenol, 4-amino-2-[(2-hydroxyethyl)amino]-methylphenol, 4-amino-2-(methoxymethyl)phenol, 4-amino-2-(2-hydroxyethyl)phenol, 5-amino-salicylic acid, 2,5-diaminopyridine, 2,4,5,6-tetraamino-pyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)-methyl]-1H-pyrazole, 1-[(4-chlorophenyl)methyl]-4,5-diamino-1H-pyrazole, 4,5-diamino-1-methyl-1H-pyrazole, 4,5-diamino-3-methyl-1-phenyl-1H-pyrazole, 4,5-diamino-1-(2-hydroxyethyl)-3-methyl-1H-pyrazole, 2-amino-phenol, 2-amino-6-methylphenol and 2-amino-5-methyl-phenol, or their salts.
(ii) Couplers: phenols, resorcinol and naphthol derivatives such as: naphthalene-1,7-diol, benzene-1,3-diol, 4-chlorobenzene-1,3-diol, naphthalen-1-ol, 2-methyl-naphthalen-1-ol, naphthalene-1,5-diol, naphthalene-2,7-diol, benzene-1,4-diol, 2-methyl-benzene-1,3-diol, 7-amino-4-hydroxy-naphthalene-2-sulfonic acid, 2-isopropyl-5-methylphenol, 1,2,3,4-tetrahydronaphthalene-1,5-diol, 2-chloro-benzene-1,3-diol, 4-hydroxy-naphthalene-1-sulfonic acid, benzene-1,2,3-triol, naphthalene-2,3-diol, 5-dichloro-2-methylbenzene-1,3-diol, 4,6-dichlorobenzene-1,3-diol, 2,3-dihydroxy-[1,4]naphthoquinone; and 1-Acetoxy-2-methylnaphthalene; m-phenylenediamines such as: 2,4-diaminophenol, benzene-1,3-diamine, 2-(2,4-diamino-phenoxy)-ethanol, 2-[(3-amino-phenyl)-(2-hydroxy-ethyl)-amino]-ethanol, 2-mehyl-benzene-1,3-diamine, 2-[[2-(2,4-diamino-phenoxy)-ethyl]-(2-hydroxy-ethyl)-amino]-ethanol, 4-{3-[(2,4-diaminophenyl)oxy]-propoxy}benzene-1,3-diamine, 2-(2,4-diamino-phenyl)-ethanol, 2-(3-amino-4-methoxy-phenylamino)-ethanol, 4-(2-amino-ethoxy)-benzene-1,3-diamine, (2,4-diamino-phenoxy)-acetic acid, 2-[2,4-diamino-5-(2-hydroxy-ethoxy)-phenoxy]-ethanol, 4-ethoxy-6-methyl-benzene-1,3-diamine, 2-(2,4-diamino-5-methyl-phenoxy)-ethanol, 4,6-dimethoxy-benzene-1,3-diamine, 2-[3-(2-hydroxy-ethylamino)-2-methyl-phenylamino]-ethanol, 3-(2,4-diamino-phenoxy)-propan-1-ol, N-[3-(dimethylamino)phenyl]urea, 4-methoxy-6-methylbenzene-1,3-diamine, 4-fluoro-6-methylbenzene-1,3-diamine, 2-({3-[(2-hydroxyethyl)amino]-4,6-dimethoxyphenyl}-amino)ethanol, 3-(2,4-diaminophenoxy)-propane-1,2-diol, 2-[2-amino-4-(methylamino)-phenoxy]ethanol, 2-[(5-amino-2-ethoxy-phenyl)-(2-hydroxy-ethyl)-amino]-ethanol, 2-[(3-aminophenyl)amino]ethanol, 2,4-Diamino-5-(2'-hydroxyethyloxy)toluene; N,N-Dimethyl-3-ureidoaniline; N-(2-aminoethyl)benzene-1,3-diamine, 4-{[(2,4-diamino-phenyl)oxy]methoxy}-benzene-1,3-diamine, 1-methyl-2,6-bis(2-hydroxyethylamino)benzene; and 2,4-dimethoxy-benzene-1,3-diamine; m-aminophenols such as: 3-amino-phenol, 2-(3-hydroxy-4-methyl-phenylamino)-acetamide, 2-(3-hydroxy-phenylamino)-acetamide, 5-amino-2-methyl-phenol, 5-(2-hydroxyethylamino)-2-methyl-phenol, 5-amino-2,4-dichloro-phenol, 3-amino-2-methyl-phenol, 3-amino-2-chloro-6-methyl-phenol, 5-amino-2-(2-hydroxy-ethoxy)-phenol, 2-chloro-5-(2,2,2-trifluoro-ethylamino)-phenol, 5-amino-4-chloro-2-methyl-phenol, 3-cyclopentylamino-phenol, 5-[(2-hydroxyethyl)amino]-4-methoxy-2-methylphenol, 5-amino-4-methoxy-2-methylphenol, 3-(dimethylamino)phenol, 3-(diethylamino)phenol, 5-amino-4-fluoro-2-methylphenol, 5-amino-4-ethoxy-2-methylphenol, 3-amino-2,4-dichloro-phenol, 3-[(2-methoxyethyl)amino]phenol, 3-[(2-hydroxyethyl)-amino]phenol, 5-amino-2-ethyl-phenol, 5-amino-2-methoxyphenol, 5-[(3-hydroxypropyl)amino]-2-methylphenol, 3-[(3-hydroxy-2-methylphenyl)-amino]propane-1,2-diol, 3-[(2-hydroxyethyl)amino]-2-methylphenol; 1-Methyl-2-hydroxy-4-(2'-hydroxyethyl)amino-benzene; 1,3-Bis-(2,4-Diaminophenoxy)propane; 1-Hydroxy-2-methyl-5-amino-6-chlorobenzene; and heterocyclic derivatives such as: 3,4-dihydro-2H-1,4-benzoxazin-6-ol, 4-methyl-2-phenyl-2,4-dihydro-3H-pyrazol-3-one, 6-methoxyquinolin-8-amine, 4-methylpyridine-2,6-diol, 2,3-dihydro-1,4-benzodioxin-5-ol, 1,3-benzodioxol-5-ol, 2-(1,3-benzodioxol-5-ylamino)ethanol, 3,4-dimethylpyridine-2,6-diol, 5-chloropyridine-2,3-diol, 2,6-dimethoxypyridine-3,5-diamine, 1,3-benzodioxol-5-amine, 2-{[3,5-diamino-6-(2-hydroxy-ethoxy)-pyridin-2-yl]oxy}-ethanol, 1H-indol-4-ol, 5-amino-2,6-dimethoxypyridin-3-ol, 1H-indole-5,6-diol, 1H-indol-7-ol, 1H-indol-5-ol, 1H-indol-6-ol, 6-bromo-1,3-benzodioxol-5-ol, 2-aminopyridin-3-ol, pyridine-2,6-diamine, 3-[(3,5-diaminopyridin-2-yl)oxy]propane-1,2-diol, 5-[(3,5-diaminopyridin-2-yl)oxy]pentane-1,3-diol, 1H-indole-2,3-dione, indoline-5,6-diol, 3,5-dimethoxypyridine-2,6-diamine, 6-methoxypyridine-2,3-diamine; 3,4-dihydro-2H-1,4-benzoxazin-6-amine; 4-hydroxy-N-methylindole, 1H-5-methylpyrazol-5-one, 1-phenyl-3-methylpyrazol-5-one, 2,6-dimethylpyrazolo[1,5-b]-1,2,4-triazole, 2,6-dimethyl[3,2-c]-1,2,4-triazole, 6-methylpyrazolo-[1,5-a]benzimidazole, 2,6-dihydroxypyridine, 2,6-dihydroxy-3,4-dimethylpyridine, 5-methylpyrazolo[5,1-e]-1,2,3-triazole, 5-methyl-6-chloropyrazolo[5,1-e]-1,2,3,-triazole, 5-phenylpyrazolo[5,1-e]-1,2,3-triazole and its addition salts, 1H-2,6-dimethylpyrazolo[1,5-b]-1,2,4-triazole tosylate, 7,8-dicyano-4-methylimidazolo-[3,2-a]imidazole, 2,7-dimethylpyrazolo-[1,5-a]pyrimidin-5-one, 2,5-dimethylpyrazolo[1,5-a]pyrimidin-7-one, and 2-methyl-5-methoxymethyl-pyrazolo[1,5-a]pyrimidin-7-one; 6-Hydroxybenzomorpholine; and 3-Amino-2-methylamino-6-methoxypyridine; 1-Phenyl-3-methyl-5-pyrazolone-2,4-dihydro-5,2-phenyl-3H-pyrazole-3-one, alone or in admixture with one another.
   Preferred coupler substances include: N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di-[(2-hydroxyethyl)amino]-1,5-dimethoxybenzene, 2,3-diamino-6-methoxypyridine, 3-amino-6-methoxy-2-(methylamino)-pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1-(3-hydroxypropoxy)benzene, 1-(2-aminoethoxy)-2,4-diaminobenzene, 2-amiono-1-(2-hydroxyethoxy)-4-methylaminobenzene, 2,4-diaminophenoxyacetic acid, 3-[di(2-hydroxyethyl)-amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di-(2,4-diaminophenoxy)methane, 1,3-diamino-2,4-dimethoxybenzene, 2,6-bis-(2-hydroxyethyl)aminotoluene, 4-hydroxyindol, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methyl-phenol, 3-amino-phenol, 2-[(3-hydroxyphenyl)amino]acetamide, 5-[(2-hydroxyethyl)amino]-2-methylphenol, 3-[(2-hydroxyethyl)amino]-phenol, 3-[(2-methoxyethyl)amino]-phenol, 5-amino-2-ethylphenol, 2-(4-amino-2-hydroxyphenoxy)-ethanol, 5-[(3-hydroxypropyl)amino]-2-methylphenol, 3-[(2,3-dihydroxypropyl)-amino]-2-methylphenol, 3-[(2-hydroxyethyl)amino]-2-methylphenol,2-amino-3-hydroxypyridine, 5-amino-4-chloro-2-methylphenol, 1-naphthol, 1,5-dihydroxy-naphthalene, 1,7-dihydroxy-naphthalene, 2,3-dihydroxynaphthalene, 2,7-dihydroxynaphthalene, 2-methyl-1-naphthol acetate, 1,3-dihydroxy-benzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxy-aniline, 5-[(2-hydroxyethyl)amino]-1,3-benzodioxole, 6-bromo-1-hydroxy-3,4-methylendioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxy-indole, 6-hydroxyindole, 7-hydroxy-indole and 2,3-indolindione, or their salts.
(iii) Compounds coupling with themselves: 2-amino-5-methylphenol; 2-amino-6-methylphenol, 2-amino-5-ethoxyphenol or 2-propylamino-5-aminopyridine.

The hair dye compositions of the present invention will generally comprise from 0.001% to 15% by weight of dye precursors. For example compositions providing low intensity dyeing such as natural blond to light brown hair shades generally comprise from 0.001 % to 7% by weight, preferably from 0.1% to 5% by weight, more preferably from about 0.2% to 2% by weight of dyeing composition of precursors and couplers. Darker shades such as browns and black typically comprise from 0.001% to 15% by weight, preferably from 0.05% to 10% by weight, more preferably form 1% to 5% by weight of precursors and couplers.
The developer substances and coupler substances are generally used in approximately equimolar amounts; however, it is not disadvantageous if the developer substances are present in this regard in a certain excess or deficit, for example a coupler:developer ratio of 1:3 to 1:0.3.

To achieve certain color shades, the agent for the oxidative coloring of keratin fibers (a) may also contain common natural and/or synthetic direct dyes, for example vegetable dyes such as henna or indigo, triphenylmethane dyes, aromatic nitro dyes, azo dyes, quinone dyes or cationic or anionic dyes.

Suitable synthetic dyes are, for example, 6-hydroxy-5-[(4-sulfophenyl)azo]-2-naphthalene sulfonic acid disodium salt (C.I. 15985; Food Yellow No. 3; FD&C Yellow No. 6), 2,4-dinitro-1-naphthol-7-sulfonic acid disodium salt (C.I.10316; Acid Yellow No. 1; Food Yellow No. 1), 2-(indan-1,3-dion-2-yl)quinoline-x,x-sulfonic acid (mixture of mono- and disolfuonic acid) (C.I.47005;D&C Yellow No. 10; Food Yellow No. 13, Acid Yellow No. 3), 5-hydroxy-1-(4-sulfophenyl)-4-[(4-sulfophenyl)azo]pyrazole-3-carboxylic acid sodium salt (C.I. 19140; Food Yellow No. 4; Acid Yellow No. 23), 9-(2-carboxyphenyl)-6-hydroxy-3H-xanthen-3-one (C.I.45350; Acid Yellow No. 73; D&C Yellow No. 8), 5-[(2,4-dinitrophenyl)amino]-2-phenylaminobenzenesulfonic acid sodium salt (C.I. 10385; Acid Orange No. 3), 4-[(2,4-dihydroxyphenyl)azo]-benzene sulfonic acid monosodium salt (C.I. 14270; Acid Orange No. 6), 4-[(2-hydroxynaphth-1-yl)azo]benzenesulfonic acid sodium salt (C.I. 15510; Acid Orange No. 7), 4-[(2,4-dihydroxy-3-[(2,4-dimethylphenyl)azo]phenyl)azo]-benzene sulfonic acid sodium salt (C.I.20170; Acid Orange No. 24), 4-hydroxy-3-[(4-sulfonaphth-1-yl)azo]-1-naphthalene sulfonic acid disodium salt (C.I. 14720; Acid Red No. 14), 6-hydroxy-5-[(4-sulfonaphth-1-yl)azo]-2,4-naphthalene-disulfonic acid trisodium salt (C.I. 16255; Ponceau 4R; Acid Red No. 18), 3-hydroxy-4-[(4-sulfonaphth-1-yl)azo]-2,7-naphthalene-disulfonic acid trisodium salt (C.I. 16185; Acid Red No. 27), 8-amino-1-hydroxy-2-(phenylazo)-3,6-naphthalene disulfonic acid disodium salt (C.I.17200; Acid Red No. 33), 5-(acetylamino)-4-hydroxy-3-[(2-methylphenyl)azo]-2,7-naphthalene disulfonic acid disodium salt (C.I.18065; Acid Red No. 35), 2-(3-hydroxy-2,4,5,7-tetraiodo-dibenzopyran-6-on-9-yl)-benzene sulfonic acid disodium salt (C.I. 45430; Acid Red No. 51), N-[6-(diethylamino)-9-(2,4-disulfophenyl)-3H-xanthen-3-yliden]-N-ethylethanaminium hydroxide, inner Salt, sodium salt (C.I.45100; Acid Red No. 52), 8-[(4-(phenylazo)phenyl)azo]-7-naphthol-1,3-disulfonic acid disodium salt (C.I. 27290; Acid Red No. 73), 2',4',5',7'-tetrabromo-3',6'-dihydroxyspiro[isobenzofuran-1-(3H),9'-[9H]xanthen]-3-one disodium salt (C.I.45380; Acid Red No. 87), 2',4',5',7'-tetrabromo-4,5,6,7-tetrachloro-3',6'-dihydroxyspiro[isobenzofuran-1(3H),9'[9H]xanthen]-3-one disodium salt (C.I. 45410; Acid Red No. 92), 3',6'-dihydroxy-4',5'-diiodospiro[isobenzofuran-1(3H),9'(9H)-xanthen)-3-one disodium salt (C.I. 45425; Acid Red No. 95), (2-sulfophenyl)-di-[4-(ethyl-((4-sulfophenyl)methyl)-amino)phenyl]carbenium disodium salt, betaine (C.I. 42090; Acid Blue No.9; FD&C Blue No.1), 1,4-bis-[(2-sulfo-4-methyl-phenyl)amino]-9,10-anthraquinone disodium salt (C.I. 61570; Acid Green No. 25), bis-[4-(dimethylamino)phenyl]-(3,7-disulfo-2-hydroxynaphth-1-yl)carbenium inner salt, monosodium salt (C.I. 44090; Food Green No. 4; Acid Green No. 50), bis-[4-(diethylamino)phenyl](2,4-disulfophenyl)carbenium inner salt, sodium salt (2:1) (C.I. 42045; Food Blue No. 3; Acid Blue No. 1), bis[4-(diethylamino)phenyl]-(5-hydroxy-2,4-disulfophenyl)carbenium inner salt, calcium salt (2:1) (C.I. 42051; Acid Blue No. 3), 1-amino-4-(cyclohexylamino)-9,10-anthraquinone-2-sulfonic acid sodium salt (C.I. 62045; Acid Blue No. 62), 2-(1,3-dihydro-3-oxo-5-sulfo-2H-indol-2-yliden)-2,3-dihydro-3-oxo-1H-indol-5-sulfonic acid disodium salt (C.I. 73015; Acid Blue No. 74), 9-(2-carboxyphenyl)-3-[(2-methylphenyl)-amino]6-[(2-methyl-4-sulfophenyl)amino]xanthylium inner salt, monosodium salt (C.I. 45190; Acid Violet No. 9), 1-hydroxy-4-[(4-methyl-2-sulfophenyl)amino]-9,10-anthraquinone sodium salt (C.I. 60730; D&C Violet No. 2; Acid Violet No. 43), bis-[3-nitro-4-[(4-phenylamino)-3-sulfophenylami no]phenyl]-sulfone (C.I. 10410; Acid Brown No. 13), 5-amino-4-hydroxy-6-[(4-nitrophenyl)azo]3-(phenylazo)-2,7-naphthalene disulfonic acid disodium salt (C.I.20470; Acid Black No. 1), 3-hydroxy-4-[(2-hydroxynaphth-1-yl)azo]-7-nitronaphthalenesulfonic acid chromium complex (3:2) (C.I. 15711; Acid Black No. 52), 3-[(2,4-dimethyl-5-sulfophenyl)azo]-4-hydroxy-1-naphthalene-sulfonic acid disodium salt (C.I. 14700; Food Red No. 1; Ponceau SX; FD&C Red No. 4), 4-(acetylamino)-5-hydroxy-6-[(7-sulfo-4-[(4-sulfophenyl)azo]naphth-1 yl)azo]-1,7-naphthalene disulfonic acid tetrasodium salt (C.I. 28440; Food Black No. 1), 3-hydroxy-4-(3-methyl-5-oxo-1-phenyl-4,5-dihydro-1H-pyrazole-4-yl-azo)-naphthalene-1-sulfonic acid sodium salt chromium complex (Acid Red No. 195).
The following are preferred cationic direct-dyeing dye compounds: 9-(dimethylamino)-benzo[a]phenoxazin-7-ium chloride (C.I. 51175; Basic Blue No. 6), di[4-(diethylamino)phenyl]-[4-(ethylamino)naphthyl]carbenium chloride (C.I. 42595; Basic Blue No. 7), 3,7-di(dimethylamino)phenothiazin-5-ium chloride (C.I. 52015; Basic Blue No. 9), di[4-dimethylamino)phenyl][4(phenylamino)naphthyl]carbenium chloride (C.I. 44045; Basic Blue No. 26), 2-[(4-(ethyl(2-hydroxyethyl)amino)phenyl)azo]-6-methoxy-3-methylbenzothiazol ium methylsulfate (C.I. 11154; Basic Blue No. 41), 8-amino-2-bromo-5-hydroxy-4-imino-6-[(3-(trimethylammonio)phenyl)amino]-1 (4H)naphthalenone chloride (C.I.56059; Basic Blue No. 99), bis-[4-(dimethylamino)phenyl][4-(methylamino)phenyl]carbenium chloride (C.I. 42535; Basic Violet No. 1), tris-(4-amino-3-methylphenyl)carbenium chloride (C.I. 425 20; Basic Violet No. 2), tris-[4-(dimethylamino)phenyl]carbenium chloride (C.I. 42555; Basic Violet No. 3), 2-[3,6-(diethylamino)dibenzopyranium-9-yl]benzoic acid chloride (C.I. 45170; Basic Violet No. 10), di(4-aminophenyl)(4-amino-3-methylphenyl)carbenium chloride (C.I. 42510; Basic Violet No. 14), 1,3-bis[(2,4-diamino-5-methylphenyl)azo]-3-methylbenzene (C.I. 21010;Basic Brown No. 4), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (C.I. 12251; Basic Brown No.17), 1-[(4-amino-3-nitrophenyl)azo]-7-(trimethylammonio)-2-naphthol chloride (C.I. 12251; Basic Brown No. 17), 3,7-diamino-2,8-dimethyl-5-phenylphenazinium chloride (C.I. 50240; Basic Red No. 2), 1,4-dimethyl-5-[(4(dimethylamino)phenyl)azo]-1,2,4-triazolium chloride (C.I. 11055; Basic Red No. 22), 2-hydroxy-1-[(2-methoxyphenyl)azo]-7-(trimethylammonio)naphthalene chloride (C.I. 12245; Basic Red No. 76), 2-[2-((2,4-dimethoxyphenyl)amino)ethenyl]-1,3,3-trimethyl-3H-indol-1-ium-chloride (C.I. 48055; Basic Yellow No. 11), 3-methyl-1-phenyl-4-[(3-(trimethylammonio)phenyl)azo]-pyrazole-5-one chloride (C.I. 12719; Basic Yellow No. 57), bis-[4-(diethylamino)phenyl]phenylcarbenium hydrogen sulfate(1:1) (C.I. 42040; Basic Green No. 1).
To improve the color balance and to produce special shades the following nonionic direct-dyeing dye compounds have proven to be useful in the compositions according to the invention: 1-amino-2-[(2-hydroxyethyl)amino]-5-nitrobenzene (HC Yellow No. 5), 1-(2-hydroxyethoxy)-2-[(2-hydroxyethyl)amino]-5-nitrobenzene (HC Yellow No. 4), 1-[(2-hydroxyethyl)amino]-2-nitrobenzene (HC Yellow No. 2), 2-[(2-hydroxyethyl)amino]-1-methoxy-5-nitrobenzene, 2-amino-3-nitrophenol, 1-(2-hydroxyethoxy)-3-methylamino-4-nitrobenzene, 2,3-(dihydroxypropoxy)-3-methylamino-4-nitrobenzene, 2-[(2-hydroxyethyl)amino]-5-nitrophenol (HC Yellow No. 11), 3-[(2-aminoethyl)amino]-1-methoxy-4-nitrobenzene hydrochloride (HC Yellow No.9), 1-[(2-ureidoethyl)amino]-4-nitrobenzene, 4-[(2,3-dihydroxypropyl)amino]-3-nitro-1-trifluoromethylbenzene (HC Yellow No. 6), 1-chloro-2,4-bis-[(2-hydroxyethyl)amino]-5-nitrobenzene (HC Yellow No. 10), 4-[(2-hydroxyethyl)amino]-3-nitro-1-methylbenzene, 1-chloro-4-[(2-hydroxyethyl)amino]-3-nitrobenzene (HC Yellow No. 12), 4-[(2-hydroxyethyl)-amino]-3-nitro-1-trifluoromethylbenzene (HC Yellow No. 13), 4-[(2-hydroxyethyl)amino]-3-nitrobenzonitrile (HC Yellow No. 14), 4-[(2-hydroxyethyl)amino]-3-nitrobenzamide (HC Yellow No. 15), 1-amino-4-[(2-hydroxyethyl)amino]-2-nitrobenzene (HC Red No. 7), 2-amino-4,6-dinitrophenol, 2-ethylamino-4,6-dinitrophenol, 4-amino-2-nitrodiphenylamine (HC Red No. 1), 1-amino-4-[di(2-hydroxyethyl)amino]-2-nitrobenzene hydrochloride (HC Red No. 13), 1 -amino-5-chloro-4-[(2hydroxyethyl)amino]-2-nitrobenzene, 4-amino-1-[(2-hydroxyethyl)-amino]-2-nitrobenzene (HC Red No. 3), 4-amino-3-nitrophenol, 4-[(2-hydroxyethyl)amino]-3-nitrophenol, 1-[(2-aminoethyl)amino]4-(2-hydroxyethoxy)-2-nitrobenzene (HC Orange No. 2), 4-(2,3-dihydroxypropoxy)-1-[(2-hydroxyethyl)amino]-2-nitrobenzene (HC Orange No. 3), 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-[(2-hydroxyethyl)-amino]4,6-dinitrophenol, 4-ethylamino-3-nitrobenzoic acid, 2-[(4-amino-2-nitrophenyl)amino]-benzoic acid, 2-chloro-6-methylamino-4-nitrophenol 2-chloro-6-[(2-hydroxyethyl)amino]4-nitrophenol, 2-chloro-6-ethylamino-4-nitrophenol, 2-amino-6-chloro-4-nitrophenol, 4-[(3-hydroxypropyl)-amino]-3-nitrophenol, 2,5-diamino-6-nitropyridine, 1,2,3,4-tetrahydro-6-nitrochinoxalin, 7-amino-3,4-dihydro-6-nitro-2H-1,4-benzoxazine (HC Red No. 14),1,4-bis-[(2-hydroxyethyl)-amino]-2-nitrobenzene, 1-(2-hydroxyethyl)ami no-2-nitro-4-[di(2-hydroxyethyl)amino]benzene (HC Blue No. 2), 1-amino-3-methyl-4-[(2-hydroxyethyl)amino]-6-nitrobenzene (HC Violet No. 1), 4-[ethyl-(2-hydroxyethyl)amino]-1-[(2-hydroxyethyl)amino]-2-nitrobenzene hydrochloride (HC Blue No. 12), 4-[di(2-hydroxyethyl)amino]-1-[(2-methoxyethyl )amino]-2-nitrobenzene (HC Blue No. 11), 1-[(2,3-dihydroxy-propyl)amino]4-[methyl-(2-hydroxyethyl)amino]-2-nitrobenzene (HC Blue No. 10), 1-[(2,3-dihydroxypropyl)amino]4-[ethyl-(2-hydroxyethyl)amino]-2-nitrobenzene hydrochloride (HC Blue No. 9), 1-(3-hydroxypropylamino)-4-[di(2-hydroxyethyl)amino]-2-nitrobenzene (HC Violet No. 2), 1-methylamino-4-[methyl-(2,3-dihydroxypropyl)amino]-2nitrobenzene (HC Blue No. 6), 2-((4-amino-2-nitrophenyl)amino)-5-dimethylamino-benzoic acid (HC Blue No. 13), 1,4-di[(2,3-dihydroxypropyl)amino]-9,10-anthraquinone, 1-[(2-hydroxyethyl)amino]4-methylamino-9,10-anthraquinone (C.I. 61505, disperse Blue No. 3), 2-[(2-aminoethyl)amino]-9,10-anthraquinone (HC Orange No. 5),1-hydroxy-4-[(4-methyl-2-sulfophenyl)amino]-9,10-anthraquinone, 1-[(3-aminopropyl)amino]4-methylamino-9,10-anthraquinone (HC Blue No. 8), 1-[(3-aminopropyl)amino]-9,10-anthraquinone (HC Red No. 8), 1,4-diamino-2-methoxy-9,10-anthraquinone (C.I.62015, disperse Red No. 11, Solvent Violet No. 26), 1,4-dihydroxy-5,8-bis[(2-hydroxyethyl)amino-9,10-anthraquinone (C.I. 62500, disperse Blue No. 7, Solvent Blue No. 69), 1-[di-(2-hydroxyethyl)amino]-3-methyl-4-[(4-nitrophenyl)azo]benzene (C.I. 11210, disperse Red No. 17), 4-[(4-aminophenyl)azo]-1-[di(2-hydroxyethyl)amino]-3-methylbenzene (HC Yellow No. 7), 2,6-diamino-3-[(pyridine-3-yl)azo]pyridine, 2-((4-(acetylamino)phenyl)-azo)-4-methylphenol (C.I. 11855; disperse Yellow No. 3).
The following compounds may also be used as direct-dyeing dye compounds: 2-amino-4,6-dinitrophenol, 2-ethylamino-4,6-dinitrophenol, 2-[(2-hydroxyethyl)-amino]4,6-dinitrophenol and dye compounds of the following general formula: 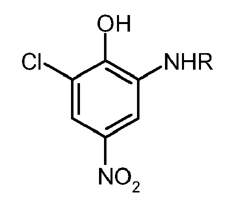 wherein R represents hydrogen, methyl, ethyl or hydroxyethyl.

The agent for the oxidative coloring of keratin fibers (a) will generally comprise from 0.001 percent to 10 percent by weight of direct dyes, preferably from 0.1 percent to 5 percent by weight, more preferably from 0.2 percent to 2 percent by weight.

Other common dyes known to be used for coloring hair and which can be contained in the colorant of the invention are described by, among others, E. Sagarin in pages 503 ff, by H. Janistyn in, "Handbuch der Kosmetika und Riechstoffe" [Handbook of Cosmetics and Fragrance Materials", vol. 3 (1973), pages 388 ff, and by K. Schrader in "Grundlagen und Rezepturen der Kosmetika" [Fundamentals and Formulations of Cosmetics], 2nd edition (1989), pages 782-815.

If the agent according to the invention is the agent for the non-oxidative coloring of keratin fibers (b) (which is free of oxidative dye precursors and oxidizing agents) it contains at least one of the aforesaid direct dyes in the aforesaid amounts.

If the agent according to the invention is the agent for the simultaneously lightening and coloring of keratin fibers (c) it comprises at least one oxidizing agent (e.g. hydrogen peroxide or persulfates) and at least one dye which is stable in the presence of the used oxidizing agent.

As oxidation-stable dyes may be especially used the following dyes: 3-(2',6'-diaminopyridyl-3'-azo)pyridine (= 2,6-diamino-3-[(pyridin-3-yl)azo]pyridine), 2-((4-eth-yl(2-hydroxyethyl)amino)-2-methylphenyl)azo-5-nitro-1,3-thiazole (Disperse Blue 106), N,N-di(2-hydroxyethyl)-3-methyl-4-[(4-nitrophenyl)azo]aniline (Disperse Red 17; C.I. 11210), 3-diethylamino-7-(4-dimethylaminophenylazo)-5-phenylphenazinium chloride (C.I. 11050), 4-(2-thiazolylazo)resorcinol, 4-[(4-phenylamino)azo]benzenesulfonic acid sodium salt (Orange IV), 1-[(3-aminopropyl)amino]-9,10-anthracenedione (HC Red No. 8), 3',3",4,5,5',5",6,7-octabromophenolsulfonphthalein (Tetrabromophenol Blue), 1-[(4-ami-no-3,5-dimethylphenyl)-(2,6-dichlorophenyl)methylene]-3,5-dimethyl-4-imino-2,5-cyclohe-xadienephosphoric acid (1:1) (Basic Blue 77), 3',3",5",5"-tetrabromo-m-cresolsulfon-phthalein, 2,4-dinitro-1-naphthol-7-sulfonic acid disodium salt (Acid Yellow 1; C.I. 10316), 4-[2'-hydroxy-1'-naphthyl)azo]benzene]sulfonic acid sodium salt (Acid Orange 7; C.I. 15510), 3',6'-dihydroxy-2',4',5',7'-tetraiodospiro[isobenzofuran-1(3H),9'(9H)xanthen]-3-one disodium salt (Acid Red 51; C.I. 45430), 6-hydroxy-5-[(2-methoxy-5-methyl-4-sulfophe-nyl)azo]-2-naphthalenesulfonic acid disodium salt (FD&C Red 40; C.I. 16035), 2,4-dini-tro-1-naphthol sodium salt (Acid Yellow 24; C.I. 10315), 2',4',5',7'-tetrabromo-4,5,6,7-tetrachloro-3',6'-dihydroxyspiro {isobenzofuran-1(3H),9'[9H]xanthen}-3-one disodium salt (Acid Red 92; C.I. 45410),4-(2-hydroxy-1-naphthylazo)-3-methylbenzenesulfonic acid so-dium salt (Acid Orange 8; C.I. 15575), 2-amino-1,4-naphthalenedione, dithizone (1,5-diphenylthiocarbazone). Nevertheless it is also possible to use one or more of the aforementioned direct dyes if they are stable against the bleaching/oxidizing agent used.

Preferred bleaching agents are inorganic perhydrate salt bleaching compounds, such as the alkali metal salts of perborates, percarbonates, perphosphates, persilicates, persulphates and the like. Especially preferred are persulphate salts, such as ammonium persulate, sodium persulfate or potassium persulfate. The bleaching agents are usually present in the agent (d) or (e) in an amount of from 5 to 65 percent by weight, preferably 20 to 50 percent by weight. The bleaching agents may especially be provided in the form of a non-aqueous cream or as a powder which is dissolved prior to use.

The agents (a), (c), (d) and (e) are used in combination with one or more known oxidants, whereas the bleaching agent (d) preferably comprises a combination of persulfates and hydrogen peroxide. Any oxidizing agent known in the art (including air-oxgen and enzyme-based oxidizing systems) may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide. These inorganic perhydrate salts may be incorporated as monohydrates, tetrahydrates etc. Alkyl and aryl peroxides, and or peroxidases may also be used. Mixtures of two or more such oxidizing agents may also be used if desired.

The oxidizing agents may be provided in aqueous solution or as a powder which is dissolved prior to use. Especially preferred for use in the compositions according to the present invention is hydrogen peroxide, alone or in combination with other oxidants.

According to the present invention the compositions comprise from 0.1% to 15% by weight, preferably from 1% to 10% by weight, and most preferably from 2% to 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably 7.5 to 9.5 more preferably pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from 0.1% to 15% by weight, preferably from 1 % to 10% by weight, and most preferably from 1 % to 8% by weight of a hydrogencarbonate ion and from 0.1 % to 10% by weight, preferably from 1 % to 7% by weight, and most preferably from 2% to 5% by weight of a source of hydrogen peroxide.

According to the present invention the composition may further optionally comprise at least one source of alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, ammonia and mixtures thereof. Particularly preferred are ammonium carbonate, ammonium carbamate, ammonium hydrogen carbonate, ammonia and mixtures thereof. The compositions of the present invention may comprise from 0.1% to 10% by weight, preferably from 0.5% to 5% by weight, most preferably from 1% to 3% by weight, of an alkalizing agent, preferably ammonium ions.

According to the present invention the compositions may further comprise a source of radical scavenger. As used herein the term radical scavenger refers to a species that can react with a reactive radical, preferably carbonate radicals, to convert the reactive radical by a series of fast reactions to a less reactive species.

Suitable radical scavengers for use herein include compounds according to the general formula (RSI):

R^{1'}-Y-C(H)(R^{3'})-R^{4'}-(C(H)(R^{5'})-Y-R^{6'})ₙ (RSI)

wherein Y is NR^{2'}, O, or S, preferably NR^{2'}, n is 0 to 2, and wherein R^{4'} is monovalent or divalent and is selected from: (a) substituted or unsubstituted, straight or branched, alkyl, mono-or polyunsaturated alkyl, heteroalkyl, aliphatic, heteroaliphatic, or heteroolefinic systems, (b) substituted or unsubstituted, mono- or polycyclic aliphatic, aryl, or heterocyclic systems, or (c) substituted or unsubstituted, mono-, poly-, or perfluoro alkyl systems; the systems of (a), (b) and (c) comprising from 1 to 12 carbon atoms and 0 to 5 heteroatoms selected from O, S, N, P, and Si; and wherein R^{4'} may be connected to R^{3'} or R^{5'} to create a 5, 6 or 7-membered ring; and wherein R^{1'}, R^{2'}, R^{3'}, R^{5'}, and R^{6'} are monovalent and are selected independently from: (a), (b) and (c) described herein above, or H.

Preferably, R^{4'} is selected from: (a) substituted or unsubstituted, straight or branched, alkyl, heteroalkyl, aliphatic, heteroaliphatic, or heteroolefinic systems, (b) substituted or unsubstituted, mono- or polycyclic aliphatic, aryl, or heterocyclic systems, or (c) substituted or unsubstituted, mono-, poly-, or perfluoro alkyl systems; more preferably R^{4'} is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or perfluoro alkyl systems; more preferably substituted or unsubstituted, straight or branched, alkyl, or heteroalkyl systems.

Preferably, the R^{4'} systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; and most preferred is O.

Preferably, R^{1'}, R^{2'}, R^{3'}, R^{5'}, and R^{6'} are selected independently from any of the systems defined for R⁴ above, and H.

In alternative embodiments, any of R^{1'}, R^{2'}, R^{3'}, R^{4'}, R^{5'}, and R^{6'} groups are substituted. Preferably, the substituent(s) is selected from: (a) the group of C-linked monovalent substituents consisting of: (i) substituted or unsubstituted, straight or branched, alkyl, mono- or polyunsaturated alkyl, heteroalkyl, aliphatic, heteroaliphatic, or heteroolefinic systems, (ii) substituted or unsubstituted, mono- or polycyclic aliphatic, aryl, or heterocyclic systems, or (iii) substituted or unsubstituted, mono-, poly-, or perfluoro alkyl systems; said systems of (i), (ii) and (iii) comprising from 1 to 10 carbon atoms and 0 to 5 heteroatoms selected from O, S, N, P, and Si; (b) the group of S-linked monovalent substituents consisting of SA¹, SCN, SO₂A¹, SO₃A¹, SSA¹, SOA¹, SO₂NA¹A², SNA¹A², and SONA¹A²; (c) the group of O-linked monovalent substituents consisting of OA¹, OCN and ONA¹A²; (d) the group ofN-linked monovalent substituents consisting ofNA¹A², (NA¹A²A³)⁺, NC, NA¹OA², NA¹SA², NCO, NCS, NO₂, N=NA¹, N=NOA¹, NA¹CN, NA¹NA²A³; (e) the group of monovalent substituents consisting of COOA¹, CON₃, CONA¹₂, CONA¹COA², C(=NA¹)NA¹A², CHO, CHS, CN, NC, and X; and (f) the group consisting fluoroalkyl monovalent substituents consisting of mono-, poly-, or perfluoro alkyl systems comprising from 1 to 12 carbon atoms and 0 to 4 heteroatoms.

For the groups (b) to (e), described above, A¹, A², and A³ are monovalent and are independently selected from: (1) H, (2) substituted or unsubstituted, straight or branched, alkyl, mono- or polyunsaturated alkyl, heteroalkyl, aliphatic, heteroaliphatic, or heteroolefinic systems, (3) substituted or unsubstituted, mono- or polycyclic aliphatic, aryl, or heterocyclic systems, or (4) substituted or unsubstituted, mono-, poly-, or perfluoro alkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.

Preferred substituents for use herein include those having a Hammett Sigma Para (σₚ) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).

Alternative suitable radical scavengers for use herein are compounds according to the general formula (RSII) : 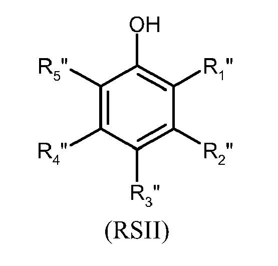 wherein R_{1"}, R_{2"}, R_{3"}, R_{4"}, and R_{5"} are each independently selected from H, COO⁻M⁺, Cl, Br, SO₃⁻M⁺, NO₂, OCH₃, OH or a C1 to C10 primary or secondary alkyl and M is either H or alkali metal. Preferably, the above-described radical scavengers have a pKa of more than 8.5 to ensure protonation of the hydroxy goup.

Other suitable radical scavengers for use herein include those selected from group (III) benzylamine, imidazole, di-tert-butylhydroxytoluene, hydroquinone, guanine, pyrazine, piperidine, morpholine, methylmorpholine, 2-methyoxyethylamine, and mixtures thereof.

Preferred radical scavengers according to the present invention are selected from the classes of alkanolamines, amino sugars, amino acids, esters of amino acids and mixtures thereof. Particularly preferred compounds are: monoethanolamine, 3-amino-l-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.

The radical scavengers according to the present invention preferably have a molecular weight of less than 500, preferably less than 300, more preferably less than 250 in order to facilitate penetration of the radical scavenger into the hair fibre. The compositions of the present invention preferably comprise from 0.1% to 10% by weight, preferably from 1% to 7% by weight of radical scavenger. The radical scavenger is also preferably selected such that it is not an identical species as the alkalizing agent. According to one embodiment of the present invention the radical scavenger may be formed insitu in the hair dyeing compositions prior to application to the hair fibres.

The compositions of the present invention may further comprise additional ingredients which include, but are not limited to, additional thickeners other than the above mentioned special anionic acrylic thickener, solvents, enzymes, surfactants, conditioning agents, carriers, antioxidants or reducing agents, stabilizers, chelants, perfumes, hair swelling agents and/or polymers. Some of these additional components are detailed hereafter.

Thickeners: The composition of the present invention may optionally further comprise at least 0.01% by weight, especially from 0.05 to 25% by weight, of thickeners. Thickeners are preferably comprised in amount sufficient to provide the composition with a viscosity of from 1 Pa.s to 40 Pa.s (1,000 to 40,000 cP) at 26 °C in order to provide a composition that can be readily applied to the hair without dripping.
The at least one thickener is chosen, for example, from:
(i) associative thickeners;
(ii) crosslinked acrylic acid homopolymers;
(iii) crosslinked copolymers of (meth)acrylic acid and of (C1-C6)alkyl acrylate;
(iv) nonionic homopolymers and copolymers containing ethylenically unsaturated monomers of ester and amide type;
(v) ammonium acrylate homopolymers and copolymers of ammonium acrylate and of acrylamide;
(vi) polysaccharides;
(vii) C12-C30 fatty alcohols and
(viii) particulate or crystalline thickeners.

(i) As used herein, the expression "associative thickener" means an amphiphilic thickener comprising both hydrophilic units and hydrophobic units, for example, at least one C8-C30 fatty chain and at least one hydrophilic unit. Representative associative thickeners that may be used are associative polymers chosen from:
   (a) nonionic amphiphilic polymers comprising at least one fatty chain and at least one hydrophilic unit;
   (b) anionic amphiphilic polymers comprising at least one hydrophilic unit and at least one fatty-chain unit;
   (c) cationic amphiphilic polymers comprising at least one hydrophilic unit and at least one fatty-chain unit; and
   (d) amphoteric amphiphilic polymers comprising at least one hydrophilic unit and at least one fatty-chain unit;

The nonionic amphiphilic polymers comprising at least one fatty chain and at least one hydrophilic unit may, for example, be chosen from:
(1) celluloses modified with groups comprising at least one fatty chain; for example: hydroxyethylcelluloses modified with groups comprising at least one fatty chain chosen from alkyl, arylalkyl and alkylaryl groups, and in which the alkyl groups are, for example, C8-C22, such as the product Natrosol Plus Grade 330 CS(C16 alkyls) sold by the company Aqualon, and the product Bermocoll EHM 100 sold by the company Berol Nobel, and celluloses modified with polyalkylene glycol alkylphenyl ether groups, such as the product Amercell Polymer HM-1500 (polyethylene glycol (15) nonylphenyl ether) sold by the company Amerchol;
(2) hydroxypropyl guars modified with groups comprising at least one fatty chain, such as the product Esaflor HM 22 (C22 alkyl chain) sold by the company Lamberti, and the products Miracare XC95-3 (C14 alkyl chain) and RE205-1 (C20 alkyl chain) sold by the company Rhodia Chimie;
(3) polyether urethanes comprising at least one fatty chain, such as C10-C30 alkyl or alkenyl groups, for instance the products Elfacos T 210 and Elfacos T 212 sold by the company Akzo or the products Aculyn 44 and Aculyn 46 and Aculyn 48 sold by the company Rohm & Haas;
(4) copolymers of vinylpyrrolidone and of fatty-chain hydrophobic monomers; examples that may be mentioned include: the products Antaron V216 and Ganex V216 (vinylpyrrolidone/hexadecene copolymer) sold by the company I.S.P., the products Antaron V220 and Ganex V220 (vinylpyrrolidone/eicosene copolymer) sold by the company I.S.P.;
(5) copolymers of C1-C6 alkyl acrylates or methacrylates and of amphiphilic monomers comprising at least one fatty chain, such as the oxyethylenated methyl methacrylate/stearyl acrylate copolymer sold by the company Goldschmidt under the name Antil 208;
(6) copolymers of hydrophilic acrylates or methacrylates and of hydrophobic monomers comprising at least one fatty chain, such as polyethylene glycol methacrylate/lauryl methacrylate copolymer; and
(7) polymers as described in WO 96/40815 comprising at least one hydrophobbic group and at least one hydrophilic group, such as the following polymers according INCI designation: Polyether-1, PEG-180/Laureth-50/TMMG Copolymer and PEG-180/Octoxynol-40/TMMG Copolymer (sold by the company United Catalysts under the name Pure-Thix 1442, Pure-Thix 1450, Pure-Thix HH, Pure-Thix L and Pure-Thix M).

The anionic amphiphilic polymers comprising at least one hydrophilic unit and at least one fatty-chain unit, may, for example, be chosen from those comprising at least one fatty-chain allyl ether unit and at least one hydrophilic unit comprising an ethylenic unsaturated anionic monomeric unit, for example, a vinylcarboxylic acid unit and further, for example, chosen from units derived from acrylic acids, methacrylic acids and mixtures thereof, wherein the fatty-chain allyl ether unit corresponds to the monomer of formula below:

CH₂=C(R¹)CH₂OBₙR (I)

in which R1 is chosen from H and CH3, B is an ethyleneoxy radical, n is chosen from zero and integers ranging from 1 to 100, R is chosen from hydrocarbon-based radicals chosen from alkyl, arylalkyl, aryl, alkylaryl and cycloalkyl radicals, comprising from 10 to 30 carbon atoms, and, further, for example, from 10 to 24 carbon atoms and even further, for example, from 12 to 18 carbon atoms.

In one embodiment, a unit of formula (I) is, for example, a unit in which R1 may be H, n may be equal to 10 and R may be a stearyl (C18) radical.
Anionic amphiphilic polymers of this type are described and prepared, according to an emulsion polymerization process, in patent EP-0 216 479 B2.
In one embodiment, anionic amphiphilic polymers are, for example, polymers formed from 20% to 60% by weight of acrylic acid and/or of methacrylic acid, from 5% to 60% by weight of lower alkyl (meth)acrylates, from 2% to 50% by weight of fatty-chain allyl ether of formula (I), and from 0% to 1% by weight of a crosslinking agent which is a well-known copolymerizable unsaturated polyethylenic monomer, for example, diallyl phthalate, allyl (meth)acrylate, divinylbenzene, (poly)ethylene glycol dimethacrylate and methylenebisacrylamide.
Examples of such polymers are crosslinked terpolymers of methacrylic acid, of ethyl acrylate and of polyethylene glycol (10 EO) stearyl ether (Steareth-10), such as those sold by the company Ciba under the names Salcare SC 80 and Salcare SC 90, which are aqueous 30% emulsions of a crosslinked terpolymer of methacrylic acid, of ethyl acrylate and of steareth-10 allyl ether (40/50/10).

The anionic amphiphilic polymers may further be chosen, for example, from those comprising at least one hydrophilic unit of unsaturated olefinic carboxylic acid type, and at least one hydrophobic unit of the type such as a (C10-C30) alkyl ester of an unsaturated carboxylic acid. The hydrophilic unit of unsaturated olefinic carboxylic acid type corresponds to, for example, the monomer of formula (II) below:

CH₂=CR¹-COOH (II)

in which R¹ is chosen from H, CH3, and C2H5, i.e. acrylic acid, methacrylic acid and ethacrylic acid units. And the hydrophobic unit of the type such as a (C10-C30) alkyl ester of an unsaturated carboxylic acid corresponds to, for example, the monomer of formula (III) below:

CH₂=CR¹-COOR² (III)

in which R¹ is chosen from H, CH3, and C2H5 (i.e. acrylate, methacrylate and ethacrylate units) and is, for example, chosen from, for example, H (acrylate units) and CH3 (methacrylate units), R² is chosen from C10-C30 alkyl radicals, for example, C12-C22 alkyl radical.
Examples of (C10-C30)alkyl esters of unsaturated carboxylic acids include lauryl acrylate, stearyl acrylate, decyl acrylate, isodecyl acrylate, and dodecyl acrylate, and the corresponding methacrylates, lauryl methacrylate, stearyl methacrylate, decyl methacrylate, isodecyl methacrylate and dodecyl methacrylate.
Anionic amphiphilic polymers of this type are disclosed and prepared, for example, according to U.S. Pat. Nos. 3,915,921 and 4,509,949.
Representative anionic amphiphilic polymers that may be used may further be chosen from polymers formed from a mixture of monomers comprising:
(1) acrylic acid, an ester of formula (IV) below:

   CH₂=CR¹-COOR² (IV)

   in which R¹ is chosen from H and CH3, R² is chosen from C10-C30 alkyl radicals, such as alkyl radicals comprising from 12 to 22 carbon atoms, and a crosslinking agent; such as polymers derived from 95% to 60% by weight of the acrylic acid (hydrophilic unit), 4% to 40% by weight of C10-C30 alkyl acrylate (hydrophobic unit), and 0% to 6% by weight of crosslinking polymerizable monomer, or polymers derived from 98% to 96% by weight of the acrylic acid (hydrophilic unit), 1% to 4% by weight of C10-C30 alkyl acrylate (hydrophobic unit) and 0.1% to 0.6% by weight of crosslinking polymerizable monomer; or
(2) acrylic acid and lauryl methacrylate, such as the polymers formed from 66% by weight of acrylic acid and 34% by weight of lauryl methacrylate.
   The crosslinking agent may be a monomer comprising a group (V) 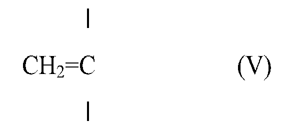 with at least one other polymerizable group whose unsaturated bonds are not conjugated with respect to one another. Mention may be made, for example, of polyallyl ethers such as polyallylsucrose and polyallyl pentaerythritol.
   Among said polymers above mention may be made, for example, of the products sold by the company Noveon under the trade names Pemulen TR1, Pemulen TR2, Carbopol 1382, and further, for example, Pemulen TR1, and the product sold by the company S.E.P.C. under the name Coatex SX.
   Suitable anionic amphiphilic fatty-chain polymers, include for example the methacrylic acid/methyl acrylate/ethoxylated alkyl dimethyl-meta-isopropenylbenzylisocyanate copolymer sold under the name Viscophobe DB 1000 by the company Amerchol.
   Further suitable anionic amphiphilic fatty-chain polymers, include polymers having the INCI designation Acrylates/Ceteth-20 Itaconate Copolymer (sold by the company National Starch under the name Structure 3001) and Acrylates/Steareth-20 Itaconate Copolymer (sold by the company National Starch under the name Structure 2001).

The cationic amphiphilic polymers used are, for example, chosen from quaternized cellulose derivatives and polyacrylates comprising amino side groups.
The quaternized cellulose derivatives are, for example, chosen from; quaternized celluloses modified with groups comprising at least one fatty chain, such as alkyl, arylalkyl and alkylaryl groups comprising at least 8 carbon atoms, and mixtures thereof, quaternized hydroxyethylcelluloses modified with groups comprising at least one fatty chain, such as alkyl, arylalkyl and alkylaryl groups comprising at least 8 carbon atoms, and mixtures thereof. Quaternized and non-quaternized polyacrylates comprising amino side groups having for example, hydrophobic groups, such as Steareth 20 (polyoxy-ethylenated(20) stearyl alcohol) and (C10-C30)alkyl PEG-20 itaconate.
The alkyl radicals borne by the above quaternized celluloses and hydroxyethylcelluloses, for example, contain from 8 to 30 carbon atoms. The aryl radicals, for example, are chosen from phenyl, benzyl, naphthyl and anthryl groups.
Examples of quaternized alkylhydroxyethyl-celluloses comprising C8-C30 fatty chains are the products Quatrisoft LM 200, Quatrisoft LM-X 529-18-A, Quatrisoft LM-X 529-18B (C12 alkyl) and Quatrisoft LM-X 529-8 (C18 alkyl) sold by the company Amerchol, and the products Crodacel QM, Crodacel QL (C12 alkyl) and Crodacel QS (C18 alkyl) sold by the company Croda.
Examples of polyacrylates comprising amino side chains is Structure Plus from the company National Starch.

Among amphoteric amphiphilic polymers comprising at least one hydrophilic unit and at least one fatty-chain unit, mention may be made, for example, of methacrylamidopropyltrimethylammonium chloride/acrylic acid/C10-C30 alkyl methacrylate copolymers, wherein the alkyl radical is, for example, a stearyl radical.
(ii) Among the crosslinked acrylic acid homopolymers that may be mentioned are those crosslinked with an allylic alcohol ether of the sugar series, such as the products sold under the names Carbopol 980, 981, 954, 2984 and 5984 by the company Noveon or the products sold under the names Synthalen M, Synthalen L and Synthalen K by the company 3V Sigma.
(iii) Crosslinked copolymers of (meth)acrylic acid and of C1-C6 alkyl acrylate may be chosen from crosslinked copolymers of methacrylic acid and of ethyl acrylate as an aqueous dispersion comprising 38% active material sold, for example, under the name Viscoatex 538C by the company Coatex, and crosslinked copolymers of acrylic acid and of ethyl acrylate as an aqueous dispersion comprising 28% active material sold under the name Aculyn 33 by the company Rohm & Haas. Crosslinked copolymers of methacrylic acid and of ethyl acrylate include an aqueous dispersion comprising 30% active material manufactured and sold under the name Carbopol Aqua SF-1 by the company Noveon.
(iv) Among the nonionic homopolymers or copolymers comprising ethylenically unsaturated monomers of ester and/or amide type, mention may be made of the products sold under the names: Cyanamer P250 by the company Cytec (polyacrylamide); PMMA MBX-8C by the company US Cosmetics (methyl methacrylate/ethylene glycol dimethacrylate copolymer); Acryloid B66 by the company Rohm & Haas (butyl methacrylate/methyl methacrylate copolymer); BPA 500 by the company Kobo (polymethyl methacrylate).
(v) Ammonium acrylate homopolymers that may be mentioned include the product sold under the name Microsap PAS 5193 by the company Hoechst.
   Copolymers of ammonium acrylate and of acrylamide include the product sold under the name Bozepol C Nouveau or the product PAS 5193 sold by the company Hoechst (which are described and prepared in documents FR-2 416 723, U.S. Pat. Nos. 2,798,053 and 2,923,692).
(vi) The polysaccharides are, for example, chosen from glucans, modified and unmodified starches (such as those derived, for example, from cereals, for instance wheat, corn or rice, from vegetables, for instance yellow pea, and tubers, for instance potato or cassaya), amylose, amylopectin, glycogen, dextrans, celluloses and derivatives thereof (methylcelluloses, hydroxyalkylcelluloses, ethyl hydroxyethylcellu loses, and carboxymethylcelluloses), mannans, xylans, lignins, arabans, galactans, galacturonans, chitin, chitosans, glucuronoxylans, arabinoxylans, xyloglucans, glucomannans, pectic acids and pectins, alginic acid and alginates, arabinogalactans, carrageenans, agars, glycosaminoglucans, succinoglucans, gum arabics, gum tragacanths, ghatti gums, karaya gums, carob gums, galactomannans, such as guar gums, and nonionic derivatives thereof (hydroxypropyl guar) and xanthan gums, and mixtures thereof. For example, suitable polysaccharides are described in "Encyclopedia of Chemical Technology", Kirk-Othmer, Third Edition, 1982, volume 3, pp. 896-900, and volume 15, pp. 439-458, in "Polymers in Nature" by E. A. MacGregor and C. T. Greenwood, published by John Wiley & Sons, Chapter 6, pp. 240-328,1980, and in "Industrial Gums-Polysaccharides and their Derivatives", edited by Roy L. Whistler, Second Edition, published by Academic Press Inc., the content of these three publications being entirely incorporated by reference.
   For example, starches, guar gums and celluloses and derivatives thereof may be used. Suitable starches include for example, of macromolecules in the form of polymers comprising elemental moieties that are anhydroglucose units. The number of these moieties and their assembly make it possible to distinguish between amylose (linear polymer) and amylopectin (branched polymer). The relative proportions of amylose and of amylopectin, and also their degree of polymerization, may vary as a function of the botanical origin of the starches. The botanical origin of the starch molecules used may be cereals or tubers. Thus, the starches may be, for example, chosen from corn starch, rice starch, cassaya starch, tapioca starch, barley starch, potato starch, wheat starch, sorghum starch and pea starch. Starches are generally in the form of a white powder, which is insoluble in cold water and which has an elementary particle size ranging from 3 to 100 microns. The starches may optionally be C1-C6 hydroxyalkylated or C1-C6 acylated (such as acetylated). The starches may also have undergone heat treatments. Distarch phosphates or of compounds rich in distarch phosphate, for instance the products sold under the references Prejel VA-70-T AGGL (gelatinized hydroxypropylated cassaya distarch phosphate) or Prejel TK1 (gelatinized cassaya distarch phosphate) or Prejel 200 (gelatinized acetylated cassaya distarch phosphate) by the company Avebe, or Structure ZEA from National Starch (hydroxypropylated corn distarch phosphate), Structure XL from National Starch (hydroxypropylated starch phosphate) may also be used.
   The guar gums may be modified or unmodified. The unmodified guar gums are, for example, the products sold under the name Vidogum GH 175 by the company Unipectine and under the names Meyro-Guar 50 and Jaguar C by the company Meyhall. The modified nonionic guar gums are, for example, modified with C1-C6 hydroxyalkyl groups. Among hydroxyalkyl groups, mention may be made, for example, of hydroxymethyl, hydroxyethyl, hydroxypropyl and hydroxybutyl groups. These guar gums are well known in the state of the art and may be prepared, for example, by reacting corresponding alkene oxides, such aspropylene oxides, with guar gum so as to obtain a guar gum modified with hydroxypropyl groups. The degree of hydroxyalkylation, which corresponds to the number of alkylene oxide molecules consumed by the number of free hydroxyl functions present on the guar gum, may, for example, range from 0.4 to 1.2.
   Such nonionic guar gums optionally modified with hydroxyalkyl groups are sold, for example, under the trade names Jaguar HP8, Jaguar HP60 and Jaguar HP120, Jaguar DC 293 and Jaguar HP 105 by the company Rhodia Chimie (Meyhall) or under the name Galactasol 4H4FD2 by the company Aqualon.
   Suitable celluloses include for example, hydroxyethylcellulose and hydroxypropylcelluloses, such as the products sold under the names Klucel EF, Klucel H, Klucel LHF, Klucel MF and Klucel G by the company Aqualon.
(vii) The C12 - C30 fatty alcohols are, for example, chosen from myristyl alcohol, cetyl alcohol, stearyl alcohol, behenyl alcohol or mixture thereof. When fatty alcohols are used as thickeners, at least one additional surfactant with HLB value above 6 is commonly included to form bi-layers with fatty alcohols. The most useful bi-layer structures include gel network phase where parallel bi-layers of fatty alcohols are swollen by water forming semi-solid creams, and vesicle dispersions where fatty alcohols bi-layers are curved into approximately spherical uni-lamellar or multi-lamellar aggregates.
(viii) The particulate and crystalline thickeners are for example, clays, fumed silica, microcrystalline cellulose, trihydroxystearin (ThixcinR), ethyleneglycol mono- and di-stearate or mixture thereof. The particulate or crystalline thickeners work via mechanisms well known in the art, for example particle aggregation, "house of card" particle arrangement or crystalline fiber formation.

Conditioning agent: The compositions of the present invention may comprise or are used in combination with a composition comprising a conditioning agent in an amount of at least 0.01 % by weight, especially from 0.1 % to 10% by weight. Conditioning agents suitable for use herein are selected from silicone materials, amino silicones, fatty alcohols, polymeric resins, polyol carboxylic acid esters, cationic polymers, cationic surfactants, insoluble oils and oil derived materials and mixtures thereof. Additional materials include mineral oils and other oils such as glycerin and sorbitol.
The conditioning agent will generally be used at levels of from 0.05% to 20% by weight of the composition, preferably of from 0.1% to 15% by weight, more preferably of from 0.2% to 10% by weight, even more preferably of from 0.2% to 2% by weight.
Particularly useful conditioning materials are cationic polymers. Conditioners of cationic polymer type may be chosen from those already know by those skilled in the art as improving at least one cosmetic properties of keratin fibres treated with a cosmetic composition. Cationic polymers may be chosen from those comprising units of at least one amine group chosen from primary, secondary, tertiary and quaternary amine groups that may either form part of the main polymer chain, or be borne by a side substituant that is directly attached to the main polymer chain. Such cationic polymers generally have a number average molecular mass ranging from 500 to 5,000,000, or more preferably from 1000 to 3,000,000. Polymers of the polyamine, polyamino amide and polyquaternary ammonium type that may be used include but are not limited to:
(1) homopolymers and copolymers derived from acrylic or methacrylic esters or amides. Copolymers of these polymers may also comprise at least one unit derived from comonomers which may be chosen from the family of acrylamides, methacrylamides, diacetone acylamides, acrylamides and methacrylicamides substituted on the nitrogen with at least one group chosen from lower (C1-C4) alkyls, acrylic and methacrylic acids and esters thereof, vinlylactams such as vinlypyrrolidone and vinylcaprolactam, and vinyl esters. Examples of such polymers include:
   Copolymers of acrylamide and of methacryloyloxyethyltrimethylammonium methosulfate, examples of which include polymers known via the INCI nomenclature as Polquaternium-5, such as the products sold under the names Reten 210, Reten 220, Reten 230, Reten 240, Reten 1104, Reten 1105, Reten 1006 by the company Hercules and Merquat 5, Merquat 5 SF by the company Nalco.
   Copolymers of vinylpyrrolidone and dimethylaminopropyl methacrylamide, examples of which include polymers known via the INCI nomenclature as Polyquaternium-28, such as the products sold under the name Gafquat HS-100 by the company International Speciality Products (ISP). Copolymers of vinyl pyrrolidone and dialkyaminoalkyl acrylates or methactylates, examples of which include polymers known via the INCI nomenclature as Polquaternium-11, such as the products sold under the name Gafquat 440, Gafquat 734, Gafquat 755, Gafquat 755N by the company International Speciality Products (ISP), and Luviquat PQ11 PM by the company BASF and Polyquat-11 SL by the company Sino Lion.
   Copolymers vinylpyrrolidone, dimethylaminopropyl methacrylamide and methacryloylaminopropyl lauryldimonium chloride, examples of which include polymers known via the INCI nomenclature as polyquaternium-55, such as the products sold under the name Styleze W-20 by the company International Speciality Products (ISP).
   Copolymers of acrylic acid, acrylamide and methacrylamidopropyltrimonium chloride, examples of which include polymers known via the INCI nomenclature as Polyquaternium-53, such as the products sold under the name Merquat 2003 by the company Nalco.
   Copolymers of dimethyaminopropylacrylate (DMAPA), acrylic acid and acrylonitrogens and diethyl sulfate, examples of which include polymers known via the INCI nomenclature as Polyquaternium-31, such as the products sold under the name Hypan QT100 by the company Lipo.
   Copolymers of acrylamide, acrylamidopropyltrimonium chloride, 2-amidopropylacrylamide sulfonate, and dimethyaminopropylacrylate (DMAPA), examples of which include polymers known via the INCI nomenclature as polyquaternium-43, such as the products sold under the name Bozequat 4000 by the company Clairant.
   Copolymers of acrylic acid, methylacrylate and methacrylamidopropyltrimonium chloride, examples of which include polymers known via the INCI nomenclature as Polyquaternium-47, such as the products sold under the name Merquat 2001 and Merquat 2001N sold commercially by Nalco.
   Copolymes of methacryloyl ethyl betaine, 2-hydroxyethyl methacrylate and methacryloyl ethyl trimethyl ammonium chloride, examples of which include polymers known via the INCI nomenclature as Polyquaternium-48, such as the products sold under the name Plascize L-450 by the company Goo Chemcial.
   Copolymers of acrylic acid diallyl dimethyl ammonium chloride and acrylamide, examples of which include polymers known via the INCI nomenclature as polyquaternium 39, such as the products sold under the name Merquat 3330 and Merquat 3331 by the company Nalco.
   Further examples include copolymers of methacrylamide methacrylamido-propyltrimonium and methacryloylethyltrimethyl ammonium chloride and their derivatives, either homo or copolymerised with other monomers, examples of which include polymers known via the INCI nomenclature as: Polyquaternium-8, Polyquaternium-9, Polyquaternium-12, Polyquaternium-13 Polyquaternium-14, Polyquaternium-15, such as the products sold under the name Rohagit KF 720 F by the company Rohm, Polyquaternium-30, such as the products sold under the name Mexomere PX by the company Chimex, Polyquaternium-33, Polyquaternium-35,
   Polyquaternium-36, such as the products sold under the name Plex 3074 L by the company Rhon, Polyquaternium 45, such as the products sold under the name Plex 3073L by the company Rohn, Polyquaternium 49, such as the products sold under the name Plascize L-440 by the company Goo Chemicals, Polyquaternium 50 such as the products sold under the name Plascize L-441 by the company Goo Chemicals, Polyquaternium-52.
(2) Cationic polysaccharides, such as cationic celluloses and cationic galactomannan gums. Among the cationic polysaccharides that maybe mentioned, for example, are cellulose ether derivatives comprising quaternary ammonium groups and cationic cellulose copolymers or cellulose derivatives grafted with a water-soluble quaternary ammonium monomer and cationic galactomannan gums. Examples include but are not limited to:
   Copolymers of hydroxyethylcelluloses and diallyldimethyl ammonium chlorides, examples of which include polymers known via the INCI nomenclature as Polyquaternium-4, such as the products sold under the name Celquat L 200 and Celquat H 100 by the company National Starch.
   Copolymers of hydroxyethylcelluloses and a trimethyl ammonium substituted epoxide, examples of which include polymers known via the INCI nomenclature as Polyquaternium-10, such as the products sold under the name AEC Polyquaternium-10 by the company A&E Connock , Catinal C-100 Catinal HC-35 Catinal HC-100 Catinal HC-200 Catinal LC-100 Catinal LC-200 by the company Toho, Celquat SC-240C Celquat SC-230M, by the company National Starch, Dekaquat 400, Dekaquat 3000 by the company Dekker, Leogard GP by the company Akzo Nobel, RITA Polyquta 400 RITA, Polyquta 3000 by the company RITA, UCARE Polymer JR-125 UCARE Polymer JR-400 UCARE Polymer JR-30M UCARE Polymer LK UCARE Polymer LR 400 UCARE Polymer LR 30M by the company Amerchol. Copolymers of hydroxyethylcelluloses and lauryl dimethyl ammonium substituted epoxides, examples of which include polymers known via the INCI nomenclature as Polyquaternium-24, such as the products sold under the name Quatrisoft polymer LM-200 by the company Amerchol. 1.
   Derivatives of Hydroxypropyl Guar, examples of which include polymers known via the INCI nomenclature as Guar Hydroxypropyltrimonium Chloride, such as the products sold under the name Catinal CG-100, Catinal CG-200 by the company Toho, Cosmedia Guar C-261N, Cosmedia Guar C-261N, Cosmedia Guar C-261N by the company Cognis, DiaGum P 5070 by the company Freedom Chemical Diamalt, N-Hance Cationic Guar by the company Hercules/Aqualon, Hi-Care 1000, Jaguar C-17, Jaguar C-2000, Jaguar C-13S, Jaguar C-14S, Jaguar Excel by the company Rhodia, Kiprogum CW, Kiprogum NGK by the company Nippon Starch.
   Hydroxypropyl derivatives of Guar Hydroxypropyltrimonium Chloride, examples of which include polymers known via the INCI nomenclature as Hydroxypropyl Guar Hydroxypropyltrimonium Chloride, such as the products sold under the name Jagaur C-162 by the company Rhodia.
(3) Polyamino amide derivatives resulting from the condensation of polyalkylene polyamines with polycarboxylic acids followed by alkylation with difunctional agents. Among the derivative, mention may be made for example to adipic acid / dimethylaminohydroxypropyl /diethylenetriamine.
(4) Polymers obtained by reaction of a polyalkylene polyamine comprising two primary amines groups and at last one secondary amine group with a decarboxylic acid chosen from diglycolic acids and saturated aliphatic dicarboxylic acids comprising from 3 to 8 carbon atoms. Nonlimiting examples of such derivatives include the adipic acid / epxoypropyl / diethylenetriamine.
(5) Cyclopolymers of dialkdiallylamine or of dialkyldiallyammonium, among which polymers mention may be made of:
   Dimethyldiallyammonium chloride polymers, examples of which include polymers known via the INCI nomenclature as Polyquaternium-6, such as the products sold under the name Merquat 100 by the company Nalco, Mirapol 100 by the company Rhodia, Rheocare CC6 by the company Cosmetic Rheologies, AEC polyquaternium-6 by the company A&E Connock, Agequat 400 by the company CPS, Conditioner P6 by the company 3V Inc., Flocare C106 by the company SNF, Genamin PDAC by the company Clariant, Mackernium 006 by the company McIntyre.
   Copolymers of acrylamides and dimethyldiallylammonium chlorides monomers, examples of which include polymers known via the INCI nomenclature as Polyquaternium-7, such as the products sold under the name AEC Polyquaternium-7 by the company A&E Connock, Agequat-5008, Agequat C-505 by the company CPS, Conditioner P7 by the company 3V Inc. Flocare C 107 by the company SNF Mackernium 007, Mackernium 007S by the company McIntyre, ME Polymer 09W by the company Toho, Merquat 550, Merquat 2200, Merquat S by the company Nalco, Mirapol 550 by the company Rhodia, Rheocare CC7, Rheocare CCP7 by the company Cosmetic Rheologies, Salcare HSP-7, Salcare SC10, Salcare Super 7 by the company Ciba. Copolymers of dimethyldiallylammoniumchlorides and acrylic acids, examples of which include polymers known via the INCI nomenclature as polyquaternary-22, such as the products sold under the name Merquat 280 and Merquat 295 by the company Nalco.
(6) Quaternary diammonium polymers comprising repeating units of formula

   [-N⁺(R¹)(R²) -A¹-N⁺(R³)(R⁴)-B¹-] [2X⁻],

   in which R¹, R², R³ and R⁴, which may be identical or different, are chosen from aliphatic, alicyclic and arylaliphatic radicals comprising from 1 to 20 carbon atoms and from lower hydroxyalkylaliphatic radicals, or R¹, R², R³ and R⁴, together or separately, constitute, with the nitrogen atoms to which they are attached, heterocycles optionally comprising a second heteroatom other then nitrogen, or R¹, R², R³ and R⁴, are choen from liner or branched C1-C6 alkyl radicals substituted with at least one group chosen from nitrile, ester, acyl and amide groups and groups of -CO-O-R5-D and -CO-NH-R5-D wherein R5 is chosen from alkylene groups and D is chosen from quaternary ammonium groups. A¹ and B¹, which may be identical or different, are chosen from linear and branched, saturated or unsaturated polymethylene groups comprising 2 to 20 carbon atoms. The polymethylene groups may comprise, linked to or intercalated in the main ring, at least one entity chosen from aromatic rings, oxygen and sulphur atoms and sulphoxide, sulphone, disulphide, amino, alkylamino, hydroxyl, quaternary, ammonium, ureido, amide and ester groups, and X- is an anion derived from inorganic and organic acids. D is chosen from a glycol residue, a bis-secondary diamine residue, a bis-primary diamine residue or a ureylene group. An examples of which include polymers known via the INCI nomenclature as Hexadimethrine chloride, where R¹, R², R³ and R⁴ are each methyl radicals, A¹ is (CH2)3 and B¹ is (CH2)6 and X = Cl. Further examples of which include polymers known via the INCI nomenclature as polyquaternium-34 where R¹ and R² are ethyl radicals and R³ and R⁴ are methyl radicals and A¹ is (CH2)3 and B¹ is (CH2)3 and X = Br, such as the products sold under the name Mexomere PAX by the company Chimax.
(7) Polyquaternary ammonium polymers comprising repeating units of formula [-N⁺(R⁶)(R⁷)-(CH2)r-NH-CO-(CH2)q-(CO)t-NH-(CH2)s-N⁺(R⁸) (R⁹)-A-] [2X-], in which R⁶, R⁷, R⁸ and R⁹ which may be identical or different, are chosen from a hydrogen atom and a methyl, ethyl, propyl, hydroxyethyl, hydroxypropyl, and -CH2CH2(OCH2CH2)pOH radicals, wherein p is equal to 0 or an integer ranging from 1 to 6, wherein R⁶, R⁷, R⁸ and R⁹ do not all simultaneously represent a hydrogen atom. R and s which maybe identical or different are each an integer ranging from 1 to 6, q is equal to 0 or an integer ranging from 1 to 34 and X- is an anion such as a halide. T is an integer chosen to be equal to 0 or 1. A is chosen from divalent radicals such as -CH2-CH2-O-CH2-CH2-. Examples of which include Polymers known via the INCI nomenclature as polyquaternium-2, where r=s=3, q=0,t=0, R⁶, R⁷, R⁸ and R⁹ are methyl groups, and A is -CH2-CH2-O-CH2-CH2, such as the products sold under the name Ethpol PQ-2 from Ethox and Mirapol A-15 by the company Rhodia.
   Polymers known via the INCI nomenclature as polyquaternium-17 where r=s=3, q=4, t=1, R⁶, R⁷, R⁸ and R⁹ are methyl groups, and A is -CH2-CH2-O-CH2-CH2.
   Polymers known via the INCI nomenclature as Polyquaternium 18, where r=s=3, q=7, t=1, R⁶, R⁷, R⁸ and R⁹ are methyl groups, and A is -CH2-CH2-O-CH2-CH2
   Polymers known via the INCI nomenclature as the block copolymer formed by the reaction of Polyquaternium-2 with Polyquaternium-17, known as Polyquaternium 27, such as the products sold under the name Mirapol 175 by the company Rhodia.
(8) Copolymers of vinylpyrrolidones and of vinylimidazoles and optionally vinylcaprolactums, examples of which include polymers known via the INCI nomenclature as Polyquaternary-16 formed from methylvinylimidazolium chlorides and vinylpyrrolidones, such as the products sold under the name Luviquat FC370, Luviquat FC550, Luviquat FC905, Luviquat HM-552 by the company BASF. Or copolymers of vinylcaprolactams and vinylpyrrolidones with methylvinylimidazolium methosulfates, examples of which include polymers known via the INCI nomenclature as Polyquaternium-46, such as the products sold under the name Luviquat Hold by the company BASF. Or copolymers of vinylpyrrolidones and quaternized imidazolines, examples of which include polymers known via the INCI nomenclature poylquaterary 44, such as the products sold under the name Luviquat Care by the company BASF
(9) Polyamines such as the product Polyquart H sold by Cognis under the reference name polyethylene glycol (15) tallow polyamine in the CTFA dictionary.
(10) Cross linked methacryloyloxy(C1-C4)alkyltri(C1-C4)alkylammonium salt polymers such as the polymers obtained by homopolymerisation of dimethylaminoethyl methacrylates quaternized with methyl chloride, or by copolymerisation of acrylamides with dimethylaminoethyl methacrylates quaternized with methyl chloride, the homo or copolymerisation being followed by crosslinking with a compound comprising olefinic unsaturation, such as methylenebisacrylamides, examples of which include polymers known via the INCI nomenclature as Polyquaternium-37, such as the products sold under the name Synthalen, CN Synthalen CR, Synthalen CU, sold by 3V sigma, or as a dispersion in another media such as the products sold under the name Salcare SC95 and Salcare SC96 by the company Ciba or Rheocare CTH(E) by the company Cosmetic Rheologies. Or in another example of which include polymers known via the INCI nomenclature as Polyquaternium-32, or when sold as a dispersion in mineral oil such as the products sold under the name Salcare SC92 by the company Ciba.
(11) Further examples of cationic polymers include polymers known via the INCI nomenclature as Polyquaternium 51, such as the products sold under the name Lipidure-PMB by the company NOF, via the INCI nomenclature as Polyquaternium 54, such as the products sold under the name Qualty-Hy by the company Mitsui, and via the INCI nomenclature as Polyquaternium 56 such as the products sold under the name Hairrol UC-4 by the company Sanyo chemicals.
(12) silicone polymers comprising cationic groups and/or groups which may be ionised into cationic groups. For example: cationic silicones of the general formula (R¹⁰-N⁺(CH3)2)-R¹¹-(Si(CH3)2-O)x-R¹¹-(N⁺(CH3)2)-R¹⁰), where R¹⁰ is an alkyl derived from coconut oil, and R¹¹ is (CH2CHOCH2O(CH2)3 and x is a number between 20 and 2000, examples of which include polymers known by the INCI nomenclature as Quaternium 80, such as the products sold under the name as Abil Quat 3272 and Abil Quat 3474 sold commercially by Goldschmidt.
   Silicones containing groups which may be ionised into cationic groups, for example aminosilicones containing at least 10 repeating siloxane -(Si(CH3)2-O) units within the polymer chain, with either terminal, graft or a mixture of terminal and graft aminofunctional groups. Example functional groups are not limited to aminoethylaminopropyl, aminoethylaminoisobutly, aminopropyl. In the case of graft polymers, the terminal siloxane units may either be (CH3)3Si-O or R¹²(CH3)2Si-O, where R¹² may be either OH or OR¹³, where R¹³ is a C1-C8 alky group, or a mixture of both functional terminal groups. These silicones are also available as preformed emulsions. Polymer with terminal siloxane units of (CH3)3Si-O examples of which include polymers known by the INCI nomenclature as trimethylsilylamodimethicone, such as the products sold under the name as DC-2-8566, DC 7224 and DC-2-8220 sold commercially by Dow Coming and SF1708 and SM 2125 sold commercially by GE Silicones and Wacker Belsil ADM 653 sold commercially by Wacker silicones. Further examples include polymers with terminal siloxane units of (R¹²O)(CH3)2Si-O where R¹² may be either OH or OR¹³, where R¹³ is a C1-C8 alky group, or a mixture of both functional terminal groups, known by the INCI nomenclature as amodimethicone, such as the products sold under the name as Wacker Belsil ADM 1100, Wacker Belsil ADM 1600, Wacker Belsil ADM 652, Wacker Belsil ADM 6057E, Wacker Belsil ADM 8020 sold commercial by Wacker Silicones, DC929, DC939 and DC949 sold commercially by Dow Coming and SM2059 sold commercially by GE silicones.
   Silicones containing groups which may be ionised into cationic groups - for example silicones containing at least 10 repeating siloxane -(Si(CH3)2-O) units within the polymer chain, with either terminal, graft or a mixture of terminal and graft aminofunctional groups, together with additional functional groups. Additional functional groups may include polyoxyalkylene, the reaction product of amines and carbinols, alky chains. For example products know by the INCI nomenclature as methoxy PEG/PPG-7/3 Aminopropyl Dimethicone, such as the product sold under the name of Abil Soft AF100 sold commercially by Degussa. For example products know by the INCI nomenclature as Bis (C13-15 Alkoxy) PG Amodimethicone, such as the product sold under the name of DC 8500 sold commercially by Dow Coming.

Surfactants: The compositions according to the present invention may further comprise one or more surfactants. Surfactants suitable for use herein generally have a lipophilic chain length of from 8 to about 30 carbon atoms and may be selected from anionic, nonionic, amphoteric and cationic surfactants and mixtures thereof. The total level of surfactant is from 0.5% to 60% by weight, preferably from 2% to 30% by weight, more preferably from 8% to 25% by weight and especially from 10% to 20% by weight.
The compositions of the invention preferably comprise a mixture of anionic and amphoteric surfactants with one or more nonionic surfactants. Anionic components, where may be present in the range of from 0.1 % to 20% by weight, preferably from 0.1 % to 15% by weight, and more preferably from 5% to 15% by weight of the composition; amphoteric or nonionic components, may independently be present is in the range from about 0.1% to 15% by weight, preferably from 0.5% to 10% by weight, more preferably from 1% to 8% by weight.
As examples of anionic surfactants, which may be used, alone or as mixtures, mention may be made, for example, of salts (such as alkaline salts, for example, sodium salts, ammonium salts, amine salts, amino alcohol salts and magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, a-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates and N-acyltaurates. The alkyl or acyl radical of all of these various compounds, for example, comprises from 8 to 24 carbon atoms, and the aryl radical, for example, is chosen from phenyl and benzyl groups. Among the anionic surfactants, which may also be used, mention may also be made of fatty acid salts such as the salts of oleic, ricinoleic, palmitic and stearic acids, coconut oil acid or hydrogenated coconut oil acid; acyl lactylates in which the acyl radical comprises from 8 to 20 carbon atoms. Weakly anionic surfactants may also be used, such as alkyl-D-galactosiduronic acids and their salts, as well as polyoxyalkylenated (C₆-C₂₄) alkyl ether carboxylic acids, polyoxyalkylenated (C₆-C₂₄) alkylaryl ether carboxylic acids, polyoxyalkylenated (C₆-C₂₄) alkylamido ether carboxylic acids and their salts, for example, those comprising from 2 to 50 ethylene oxide groups, and mixtures thereof. Anionic derivatives of polysaccharides, for example carboxyalkyl ether of alkyl polyglucosides, may be also used.
The nonionic surfactants are compounds that are well known (see, for example, in this respect "Handbook of Surfactants" by M. R. Porter, published by Blackie & Son (Glasgow and London), 1991, pp. 116-178). They may be chosen, for example, from polyethoxylated, polypropoxylated and polyglycerolated fatty acids, alkyl phenols, α-diols and alcohols comprising a fatty chain comprising, for example, from 8 to 18 carbon atoms, it being possible for the number of ethylene oxide or propylene oxide groups to range, for example, from 2 to 200 and for the number of glycerol groups to range, for example, from 2 to 30. Mention may also be made of copolymers of ethylene oxide and of propylene oxide, condensates of ethylene oxide and of propylene oxide with fatty alcohols; polyethoxylated fatty amides preferably having from 2 to 30 mol of ethylene oxide and their momoethanolamine and diethanolamine derivatives, polyglycerolated fatty amides, for example, comprising on average from 1 to 5, and such as from 1.5 to 4, glycerol groups; polyethoxylated fatty amines such as those containing from 2 to 30 mol of ethylene oxide; oxyethylenated fatty acid esters of sorbitan having from 2 to 30 mol of ethylene oxide; fatty acid esters of sucrose, fatty acid esters of polyethylene glycol, alkylpolyglycosides,
N-alkylglucamine derivatives, amine oxides such as (C₁₀-C₁₄)alkylamine oxides or N-acylaminopropylmorpholine oxides.

The amphoteric surfactants may be chosen, for example, from aliphatic secondary and tertiary amine derivatives in which the aliphatic radical is chosen from linear and branched chains comprising from 8 to 22 carbon atoms and comprising at least one water-soluble anionic group (for example carboxylate, sulphonate, sulphate, phosphate or phosphonate); mention may also be made of (C₈-C₂₀)alkylbetaines, sulphobetaines, (C₈-C₂₀)alkylamido(C₁-C₆)alkylbetaines or (C₈-C₂₀)alkylamido(C₁-C₆)alkylsulphobetaines.
Among the amine derivatives, mention may be made of the products sold under the name Miranol, as described, for example, in U.S. Pat. Nos. 2,528,378 and 2,781,354 and having the structures of:

R²-CON HCH₂CH₂-N⁺(R³)(R⁴)(CH₂COO⁻) (VI)

in which: R² is chosen from alkyl radicals derived from an acid R₂-COOH present in hydrolysed coconut oil, and heptyl, nonyl and undecyl radicals, R³ is a β-hydroxyethyl group and R⁴ is a carboxymethyl group; and of R⁵-CONHCH₂CH₂-N(B)(C) (VII)
wherein B represents -CH₂CH₂OX', C represents -(CH₂)_{z}-Y', with z=1 or 2, X' is chosen from the -CH₂CH₂-COOH group and a hydrogen atom, Y' is chosen from -COOH and -CH₂-CHOH-SO₃H radicals, R⁵ is chosen from alkyl radicals of an acid R⁵-COOH present in coconut oil or in hydrolysed linseed oil, alkyl radicals, such as C₇, C₉, C₁₁ and C₁₃ alkyl radicals, a C₁₇ alkyl radical and its iso form, and unsaturated C₁₇ radical. These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid. Salts of diethyl aminopropyl cocoaspartamid may be also used.
The cationic surfactants may be chosen from: A) the quaternary ammonium salts of general formula (VIII):

[NR¹R²R³R⁴]⁺ X⁻ (VIII)

wherein X⁻ is an anion chosen from halides (chloride, bromide and iodide), (C₂-C₆)alkyl sulphates, such as methyl sulphate, phosphates, alkyl and alkylaryl sulphonates, and anions derived from organic acids, such as acetate and lactate, and
(i) the radicals R¹ to R³, which may be identical or different, are chosen from linear and branched aliphatic radicals comprising from 1 to 4 carbon atoms, and aromatic radicals such as aryl and alkylaryl. The aliphatic radicals may comprise at least one hetero atom such as oxygen, nitrogen, sulphur and halogens. The aliphatic radicals are chosen, for example, from alkyl, alkoxy and alkylamide radicals, R⁴ is chosen from linear and branched alkyl radicals comprising from 16 to 30 carbon atoms.
   The cationic surfactant is, for example, a behenyltrimethylammonium salt (for example a chloride or bromide).
(ii) the radicals R¹ and R², which may be identical or different, are chosen from linear and branched aliphatic radicals comprising from 1 to 4 carbon atoms, and aromatic radicals such as aryl and alkylaryl. The aliphatic radicals may comprise at least one hetero atom such as oxygen, nitrogen, sulphur and halogens. The aliphatic radicals are chosen, for example, from alkyl, alkoxy, alkylamide and hydroxyalkyl radicals comprising from 1 to 4 carbon atoms; R₃ and R₄, which may be identical or different, are chosen from linear and branched alkyl radicals comprising from 12 to 30 carbon atoms, the said alkyl radicals comprise at least one function chosen from ester and amide functions.
   R³ and R⁴ are chosen, for example, from (C₁₂-C₂₂)alkylamido(C₂-C₆)alkyl and (C₁₂-C₂₂)alkylacetate radicals.
   The cationic surfactant is, for example, a dicetyldimethyl ammonium salt (for example chloride); B) the quaternary ammonium salts of imidazolinium, such as that of formula (IX) below: 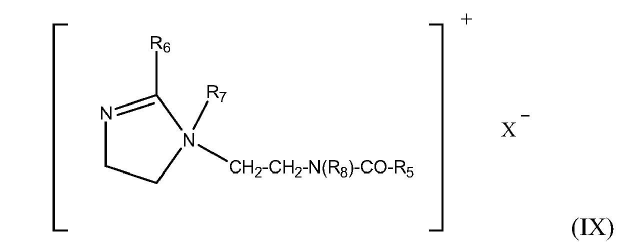
in which R5 is chosen from alkenyl and alkyl radicals comprising from 8 to 30 carbon atoms, for example fatty acid derivatives of tallow,
R6 is chosen from a hydrogen atom, C₁-C₄ alkyl radicals and alkenyl and alkyl radicals comprising from 8 to 30 carbon atoms, R7 is chosen from C₁-C₄ alkyl radicals, R8 is chosen from a hydrogen atom and C₁-C₄ alkyl radicals, and X⁻ is an anion chosen from halides, phosphates, acetates, lactates, alkyl sulphates, alkyl sulphonates and alkylaryl sulphonates.
In one embodiment, R5 and R6 are, for example, a mixture of radicals chosen from alkenyl and alkyl radicals comprising from 12 to 21 carbon atoms, such as fatty acid derivatives of tallow, R7 is methyl and R8 is hydrogen. Such a product is, for example, Quaternium-27 (CTFA 1997) or Quaternium-83 (CTFA 1997), which are sold under the names "Rewoquat®" W75, W90, W75PG and W75HPG by the company Witco,
C) the diquaternary ammonium salts of formula (X):

[R11R10R9N-(CH2)3-NR12R13R14]⁺⁺ (X)

in which R9 is chosen from aliphatic radicals comprising from 16 to 30 carbon atoms,
R10, R11, R12, R13 and R14, which may be identical or different, are chosen from hydrogen and alkyl radicals comprising from 1 to 4 carbon atoms, and
X⁻ is an anion chosen from halides, acetates, phosphates, nitrates and methyl sulphates. Such diquaternary ammonium salts, for example, include propanetallowdiammonium dichloride; and
D) the quaternary ammonium salts comprising at least one ester function, of formula (XI) below: 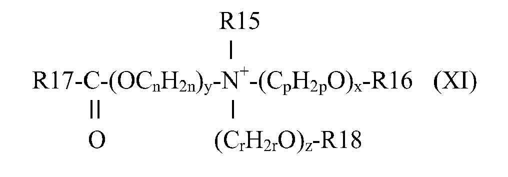 in which:
R15 is chosen from C1-C6 alkyl radicals and C1-C6 hydroxyalkyl and dihydroxyalkyl radicals; R16 is chosen from: a radical R19C(O)-, linear and branched, saturated and unsaturated C1-C22 hydrocarbon-based radicals R20, and a hydrogen atom, R18 is chosen from: a radical R21C(O)-, linear and branched, saturated and unsaturated C1-C6 hydrocarbon-based radicals R22, and a hydrogen atom, R17, R19 and R21, which may be identical or different, are chosen from linear and branched, saturated and unsaturated C7-C21 hydrocarbon-based radicals; n, p and r, which may be identical or different, are chosen from integers ranging from 2 to 6; y is chosen from integers ranging from 1 to 10; x and z, which may be identical or different, are chosen from integers ranging from 0 to 10; X-is an anion chosen from simple and complex, organic and inorganic anions; with the proviso that the sum x+y+z is from 1 to 15, that when x is 0, then R16 is R20 and that when z is 0, then R18 is R22.
In one embodiment, the ammonium salts of formula (XV) may be used, in which:
R15 is chosen from methyl and ethyl radicals, x and y are equal to 1; z is equal to 0 or 1; n, p and r are equal to 2; R16 is chosen from: a radical R19C(O)-,methyl, ethyl and C14-C22 hydrocarbon-based radicals, and a hydrogen atom; R17, R19 and R21, which may be identical or different, are chosen from linear and branched, saturated and unsaturated C7-C21, hydrocarbon-based radicals; R18 is chosen from: a radical R21C(O)- and a hydrogen atom. Such compounds are sold, for example, under the names Dehyquart by the company Cognis, Stepanquat by the company Stepan, Noxamium by the company Ceca, and Rewoquat WE 18 by the company Rewo-Witco.

Chelants: According to the present invention the compositions may comprise chelants. Chelants are well known in the art and refer to a molecule or a mixture of different molecules each capable of forming a chelate with a metal ion. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996) both incorporated herein by reference.
Chelants suitable for use herein are carboxylic acids (in particular aminocarboxylic acids), polyamines, phosphonic acids (in particular aminophosphonic acids) and polyphosphoric acids (in particular linear polyphosphoric acids), their salts and derivatives.
Examples of preferred chelants include ethylenediaminotetraacetic acid (EDTA), nitrilotriacetic acid, diethylenetriamine pentaacetic acid (DTPA), ethylenediamine disuccinic acid (EDDS), ethylenediamine diglutaric acid (EDGA), 2-hydroxypropylenediamine disuccinic acid (HPDS), glycinamide-N,N'-disuccinic acid (GADS), ethylenediamine-N-N'-diglutaric acid (EDDG), 2-hydroxypropylenediamine-N-N'-disuccinic acid (HPDDS), salts thereof and derivatives thereof.
Chelants may be incorporated into the composition of the present invention as stabilizers and or preservatives. In addition it has also been found that chelants provide hair fibre damage benefits and thus they may be utilized in order to further improve the hair damage profile of the present invention. Levels of chelants in the present invention may be as low as 0.1 % by weight, preferably at least 0.25% by weight, more preferably 0.5% by weight, for the most effective chelants such as diamine-N,N'-dipolyacid chelants and monoamine monoamide-N,N'-dipolyacid chelants (for example EDDS). Less effective chelants will be more preferably used at levels of at least 1% by weight, even more preferably above 2% by weight of the composition, depending of the efficiency of the chelant. Levels as high as 10% by weight may be used, but above this level significant formulation issues may arise.

Solvents: Suitable solvents for use in the compositions of the present invention include, but are not limited to, water, butoxydiglycol, 1,2-propylene glycol, glycerol, lower aliphatic alcohols (e.g. ethanol, n-propanol or isoproanol), ethoxydiglycol, isopropylalcohol, hexylene glycol, benzyl alcohol and dipropylene glycol.

Antioxidants or reducing agents: Suitable antioxidants or reducing agents for use in the compositions of the present invention include, but are not limited to, ascorbic acid, thioglycolic acid and sodium sulfite.

The pH of the agent for the non-oxidative coloring of keratin fibers based on direct dyes, the pH of the colorant of the invention is in the range from 5 to 10 and preferably from 6 to 9, whereas the pH of the agent for the oxidative coloring of keratin fibers based on oxidation dye precursors is from 6 to 12 and preferably from 9 to 11. The pH of the ready-to-use oxidation hair colorant (namely of the mixture of the hair colorant of the invention and the oxidant) and the reday-to-use agent for the simultaneously lightening and coloring of keratin fibers is from 5,5 to 10 and preferably from 6 to 9. The pH of the ready-to-use agent for the bleaching of keratin fibers is from 8 to 12 and preferably from 9 to 11.5.
Depending on the composition and the desired pH, the pH is preferably adjusted with ammonia or an organic amine, for example glucamine, aminomethylpropanol, monoethanolamine or triethanolamine, with an inorganic base, for example sodium hydroxide, potassium hydroxide, sodium carbonate or calcium hydroxide, or with an organic or inorganic acid, for example lactic acid, citric acid, acetic acid or phosphoric acid.

The ready-to-use compositions may be, for example, a solution, especially an aqueous or aqueous-alcoholic solution. However a particularly preferred form of the composition according to the invention is a cream, a gel or an emulsion. Its composition comprises a mixture of the dye and/or lightening/bleaching ingredients with the conventional additive ingredients usually used in this type of preparation.

According to a preferred embodiment of the present invention dye powders or dye granules or bleaching powders or bleaching granules are mixed with an appropriate carrier mass prior to use to form the ready-to-use composition. Particularly preferred is the use of pellets as described in WO 2005/044208 which are mixed with an appropriate carrier mass prior to use to form the ready-to-use composition. WO 2005/044208 herewith is explicitly incorporated herein by reference.

To attain simultaneous lightening and coloring of the fibers, the dye-containing pellets of the invention can also contain an ammonium carbonate, for example ammonium hydrogen carbonate, or an amino acid or a salt thereof, for example sodium glycinate.

Depending on the intended use, the composition (a), (b), (c), (d) or (e) according to the invention can be used with one or more oxidizing agents (lightening/bleaching; oxidation colorants) or without an oxidizing agent (non-oxidative colorants).

When used for the oxidative dyeing of keratin fibers, the afore described composition (a) is mixed with an oxidant just before use, and an amount of the ready-to-use mixture sufficient for dyeing, as a rule 60 to 200 grams, is applied to the fibers.
If the colorant of the invention contains no oxidation dye precursors or if it contains oxidation dye precursors that are readily oxidized by atmospheric oxygen, said colorant can be applied to the keratin fibers directly without previous mixing with an oxidant.
Suitable oxidants for developing the coloration are described above, whereas a hydrogen peroxide, or its addition compounds with urea, melamine or sodium bromate, in the form of a 1 to 12 percent by weight, preferably 6 percent by weight, aqueous solution, is preferred as the oxidizing agent. The mixing ratio of composition (a) to oxidant depends on the concentration of the oxidant and as a rule is 5:1 to 1:2 and preferably 1:1, the amount of oxidant in the ready-to-use composition being preferably 0.5 to 8 weight percent and particularly 1 to 4 weight percent. The ready-to-use colorant is allowed to act on the keratin fibers (for example human hair) at 15 °C to 50 °C for 10 to 45 minutes and preferably for 15 to 30 minutes after which the fibers are rinsed with water and dried. Optionally, following this rinsing, the keratin fibers are washed with a shampoo and possibly post-rinsed with a weak organic acid, for example tartaric acid. The keratin fibers are then dried.

The agent for the simultaneously lightening and coloring of keratin fibers (c) or (e) as well as the agent for the bleaching of keratin fibers (d) is normally used in the form of multi-component-kits, especially two-component-kits, wherein one component contains the oxidant and the other contains the dyes or the bleaching agents. Prior to use the components are mixed with each other and an amount of the ready-to-use mixture sufficient for coloring and/or lightening/bleaching the keratin fibers, as a rule 60 to 200 grams, is applied to the fibers. The ready-to-use composition is allowed to act on the keratin fibers (for example human hair) at 15 °C to 50 °C for 10 to 45 minutes and preferably for 15 to 30 minutes after which the fibers are rinsed with water and dried. Optionally, following this rinsing, the keratin fibers are washed with a shampoo and possibly post-rinsed with a weak organic acid, for example tartaric acid. The keratin fibers are then dried.

If necessary, the viscosity of the ready-to-use compositions (a), (b), (c) or (d) can be increased by adding an appropriate amount of the above described anionic acrylic thickener of the invention to the composition. It is also possible to thicken oxidative or non-oxidative colorants or agents for simultaneously lightening and dyeing keratin fibers or agents for bleaching keratin fibers by addition of the above described anionic acrylic thickener of the invention just prior to use. This provides simpler and more economical basic formulations.

The above described compositions (a), (b), (c) or (d) meet the requirements in terms of adhesion properties, application characteristics and viscosity adjustment in outstanding manner and are clearly easier to apply. Moreover, the above described composition s (a), (b), (c) or (d) have a uniform consistency and are cosmetically appealing. Particularly notable is the very good viscosity and outstanding stability of the above described composition s (a), (b), (c) or (d) and their excellent adhesion to hair. Furthermore, the use of the above described compositions (a), (c) or (d) makes it possible to vary the weight ratio of colorant to oxidant over a wide range (for example from 1:1 to 1:2.5) without adversely affecting the viscosity and adhesion properties of the ready-to-use oxidation colorant in an appreciable manner.

The following examples are intended to explain the subject matter of the invention more closely without limiting its scope.

### EXAMPLES

### Example 1: Oxidation Hair Colorant, Liquid

| | |
|---|---|
| 0.5000 g | ViscUp®EZ of Arch Personal Care Products, South Plainfield, USA ¹⁾ |
| 5.0000 g | 2-octyl-1-dodecanol (Eutanol G, supplied by Cognis/Germany) |
| 15.0000 g | oleic acid |
| 10.0000 g | sodium lauryl alcohol diethylene glycol ether sulfate (28% aqueous solution) |
| 1.3620 g | 4-aminophenol 1 |
| 0.5000 g | 1-naphthol |
| 0.0136 g | resorcinol |
| 0.0034 g | 2-amino-6-chloro-4-nitrophenol |
| 12.0000 g | ammonia, 25% aqueous solution |
| 1.0000 g | disodium ethylenediaminetetraacetate |
| 1.0000 g | ascorbic acid |
| 15.0000 g | isopropanol |
| ad 100.0000 g | water |

Just before use, 50 g of the foregoing hair colorant was mixed with 50 g of a 6% aqueous hydrogen peroxide solution. This gave a homogeneous, cosmetically appealing, optimally thickened, colorant formulation. The mixture thus obtained was then applied to naturally blond hair. After an exposure time of 30 min at 40 °C., the hair was rinsed with water and dried. The hair showed a bright copper-red coloration.

### Example 2: Oxidation Hair Colorant in Gel Form

| | |
|---|---|
| **Component (A)**: | Liquid Dye Carrier Composition |
| 1.20 g | ViscUp®EZ of Arch Personal Care Products, South Plainfield, USA ¹⁾ |
| 10.00 g | of lauryl alcohol |
| 6.00 g | nonylphenol ethoxylated with 4 moles of ethylene oxide |
| 6.00 g | oleic acid |
| 0.62 g | 2-(methoxymethyl)-benzene-1,4-diamine |
| 0.28 g | resorcinol |
| 5.00 g | sodium lauryl alcohol diethylene glycol ether sulfate, 28% aqueous solution |
| 1.00 g | disodium ethylenediaminetetraacetate |
| 18.00 g | ammonia, 25% aqueous solution |
| 8.00 g | ethanol |
| ad 100.00 g | water |
| **Component (B):** | Hydrogen Peroxide Emulsion |
| 10.0 g | cetylstearyl alcohol |
| 1.5 g | cholesterol |
| 4.0 g | sodium lauryl alcohol diethylene glycol ether sulfate, 28% aqueous solution |
| 35.0 g | hydrogen peroxide, 35% aqueous solution |
| 0.3 g | perfume |
| ad 100.0 g | water |

Before use, 40 g of the liquid dye carrier composition (A) was mixed with 80 g of the hydrogen peroxide emulsion (B), corresponding to an (A):(B) mixing ratio of 1:2, and 120 g of this mixture was applied to gray human hair. After an exposure time of 20 min at room temperature, the hair was rinsed with water and dried. The hair treated in this manner was colored uniformly brown from hairline to hair tips. The colorant of the invention was easily applied and did not run off the hair.

### Example 3: Oxidation Hair Colorant in Cream Form

| | |
|---|---|
| 0.40 g | sodium acrylate/sodium acryloyldimethyl taurate copolymer |
| 3.00 g | oleyl alcohol |
| 15.00 g | cetyl alcohol |
| 3.50 g | sodium lauryl alcohol diethylene glycol ether sulfate (28% aqueous solution) |
| 3.00 g | monoethanolamine |
| 1.30 g | 1-methyl-2,5-diaminobenzene |
| 1.00 g | beeswax |
| 0.65 g | resorcinol |
| 0.50 g | keratin hydrolyzate |
| 0.50 g | silk protein hydrolyzate |
| 0.50 g | 2-amino-6-chloro-4-nitrophenol |
| 0.30 g | ascorbic acid |
| ad 100.00 g | water |

Just before use, 50 g of the foregoing hair colorant was mixed with 50 g of 12% aqueous hydrogen peroxide solution. The resulting mixture was then applied to naturally blond hair. After an exposure time of 30 min at 40 °C., the hair was rinsed with water and dried. A uniform, strong brown shade was obtained.

### Example 4: Hair Tinting Agent (Non-Oxidative)

| | |
|---|---|
| 2.5 g | ViscUp®EZ of Arch Personal Care Products, South Plainfield, USA¹⁾ |
| 6.0 g | lauryl alcohol |
| 5.0 g | sodium lauryl sulfate |
| 1.5 g | 2-amino-6-chloro-4-nitrophenol |
| 1.0 g | monoethanolamine |
| 1.0 g | beeswax |
| 0.5 g | keratin hydrolyzate |
| 0.3 g | silk protein hydrolyzate |
| 0.2 g | glycine |
| ad 100.0 g | water |

A slightly gelled colorant composition was obtained which because of its outstanding viscosity characteristics was easily and uniformly applied and adhered well to the hair. After an exposure time of 20 min at 20 °C., the hair was rinsed with luke-warm water, styled and dried. The hair treated in this manner showed a uniform, very lustrous gold-orange coloration.

### Example 5: Oxidative Colorant

| | |
|---|---|
| **Component (A)**: | Dye Carrier Composition |
| 4.0 g | ViscUp®EZ of Arch Personal Care Products, South Plainfield, USA¹⁾ |
| 8.0 g | 2-octyl-1-dodecanol (Eutanol G, supplied by Cognis/Germany) |
| 3.0 g | sodium lauryl alcohol diethylene glycol ether sulfate (28% aqueous solution) |
| 2.8 g | 2,5-diaminotoluene sulfate |
| 1.0 g | resorcinal |
| 0.4 g | m-aminophenol |
| 0.2 g | 2-amino-4-(2'-hydroxyethylamino)anisole sulfate |
| 0.3 g | ascorbic acid |
| 0.1 g | ethylenediaminetetraacetic acid |
| 12.2 g | ammonia, 25% aqueous solution |
| 2.0 g | ethanol |
| ad 100.0 g | water |
| **Component (B):** | Hydrogen Peroxide - Emulsion |
| 10.0 g | cetylstearyl alcohol |
| 1.5 g | cholesterol |
| 4.0 g | sodium lauryl alcohol diethylene glycol ether sulfate, 28% aqueous solution |
| 17.0 g | hydrogen peroxide, 35% aqueous solution |
| 0.3 g | perfume |
| ad 100.0 g | water |

Before use, 40 g of the liquid dye carrier composition (A) was mixed with 80 g of the hydrogen peroxide emulsion (B), corresponding to an (A):(B) mixing ratio of 1:2, and 120 g of this mixture was applied to human hair. After an exposure time of 20 min at room temperature, the hair was rinsed with water and dried. The hair treated in this manner was of a uniform, dark-brown shade. The colorant of the invention adhered very well to the hair without running off.

### Example 6: Colorant for Simultaneously Lightening and Coloring Hair

| | |
|---|---|
| **Component (A):** | Dye Carrier Composition |
| 4.0 g | ViscUp®EZ of Arch Personal Care Products, South Plainfield, USA¹⁾ |
| 2.0 g | 4-dimethylaminophenylazo-2N-methyl-5N-methyl-imidazolium chloride |
| 8.0 g | 2-octyl-1-dodecanol (Eutanol G, supplied by Cognis/Germany) |
| 3.0 g | sodium lauryl alcohol diethylene glycol ether sulfate (28% aqueous solution) |
| 8.0 g | ammonia, 25% aqueous solution |
| 0.1 g | ethylenediaminetetraacetic acid |
| 2.0 g | ethanol |
| ad 100.0 g | water |
| **Component (B):** | Hydrogen Peroxide - Emulsion |
| 10.0 g | cetylstearyl alcohol |
| 1.5 g | cholesterol |
| 4.0 g | sodium lauryl alcohol diethylene glycol ether sulfate, 28% aqueous solution |
| 17.0 g | hydrogen peroxide, 35% aqueous solution |
| 0.3 g | perfume |
| ad 100.0 g | water |

Before use, 40 g of the liquid dye carrier composition (A) was mixed with 40 g of the hydrogen peroxide emulsion (B), corresponding to an (A):(B) mixing ratio of 1:1, and this mixture was applied to human hair. After an exposure time of 10 min at room temperature, the hair was rinsed with water and dried. The colorant of the invention adhered very well to the hair without running off.

### Example 7: Colorant for Simultaneously Lightening and Coloring Hair

| | |
|---|---|
| **Lightening Powder (A):** | |
| 20.0 g | potassium persulfate |
| 30.0 g | ammonium persulfate |
| 24.0 g | sodium silicate |
| 12.5 g | magnesium oxide |
| 6.0 g | soap beads |
| 2.0 g | dispersed silicic acid |
| 0.5 g | ethylenediaminetetraacetic acid disodium salt |
| 0.1 g | Acid Red 92 (CI 45410) |
| 0.2 g | Acid Yellow 1 (CI 10316) |
| 0.8 g | Acid Yellow 24 (CI 10315) |
| 1.0 g | Acid Orange No 8 (CI 15575) |
| 1.0 g | Acid Orange No 7 (CI 15510) |
| **Thickener (B):** | |
| 100,00 g | ViscUp®EZ of Arch Personal Care Products, South Plainfield, USA¹) |

Before use, 40 g of the above lightening powder (A) was mixed with 80 g of a 12% aqueous hydrogen peroxide solution (C) and 5 g of the above thickener (B) to form a homogeneous composition. This mixture was applied to dry human hair. After an exposure time of 20 min at 40 °C, the hair was rinsed with warm water and dried. The hair treated in this manner was of a uniform, bright orange shade. The colorant of the invention adhered very well to the hair without running off.

### ¹⁾ ViscUp®EZ is a mixture of 35-55% Sodium Acrylate/Sodium Acryloyldimethyl Taurate Copolymer and 23-35% Hydrogenated Polydecene and 20-30% Sorbitan Laurate and 5-10% Trideceth-6

Unless otherwise indicated, all percentages in the present patent application are by weight.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An agent for the oxidative coloring of keratin fibers, comprising at least one oxidative dye precursor, **characterized in that** it comprises at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer).

2. An agent for the non-oxidative coloring of keratin fibers, comprising at least one direct dye, **characterized in that** it comprises at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer).

3. An agent for the simultaneously lightening and coloring of keratin fibers, comprising at least one bleaching agent and at least one dye which is stable in the presence of the used oxidizing agent, **characterized in that** it comprises at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer).

4. An agent for the bleaching of keratin fibers, comprising at least one bleaching agent, **characterized in that** it comprises at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer).

5. An agent for the simultaneously lightening and coloring of keratin fibers, which is mixed with an oxidizing agent prior to use, comprising at least one dye which is stable in the presence of the used oxidizing agent, **characterized in that** it comprises at least one anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer).

6. An agent according to one of claims 1 to 5, **characterized in that** the monomer performing a weak acid function is selected from the group consisting of acrylic acid, methacrylic acid, itaconic acid, crotonic acid, maleic acid and fumaric acid.

7. An agent according to one of claims 1 to 6, **characterized in that** the monomer performing a strong acid function is selected from the group consisting of monomers having a function of the sulfonic acid type or phosponic acid type, such as 2-acrylamido-2-methylpropane sulfonic acid.

8. An agent according to one of claims 1 to 7, **characterized in that** the anionic acrylic polymer is crosslinkled or branched by a crosslinking agent which is selected from the group consisting of methylene bisacrylamide, ethylene glycol diacrylate, polyethylene glycol dimethacrylate, diacrylamide, cyanomethacrylate, vinyloxyethacrylate, methacrylate, formaldehyde, glyoxal, compositions of the glycidylether type and epoxydes.

9. An agent according to one of claims 1 to 8, **characterized in that** the anionic acrylic thickener is a Sodium Acrylate/Sodium Acryloyldimethyl Taurate Copolymer.

10. An agent according to claim 9, **characterized in that** the anionic acrylic thickener is Sodium Acrylate/Sodium Acryloyldimethyl Taurate Copolymer & Hydrogenated Polydecene & Sorbitan Laurate & Trideceth-6.

11. An agent according to one of claims 1 to 10, **characterized in that** the anionic acrylic thickener in an amount from 0.01 to 8.0 percent by weight (based on the active substance).

12. An agent according to one of claims 1 to 11, **characterized in that** the keratin fiber is human hair.

13. Use of an anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer), for the thickening of compositions for the oxidative or non-oxidative dyeing of keratin fibers, compositions for the simultaneously lightening and coloring of keratin fibers or compositions for the bleaching of keratin fibers.

14. A method for thickening compositions for the oxidative or non-oxidative dyeing of keratin fibers, compositions for the simultaneously lightening and coloring of keratin fibers or compositions for the bleaching of keratin fibers, **characterized in that** an appropriate amount of an anionic acrylic thickener obtained by polymerization of (i) 5 to 95 molar percent of at least one monomer performing a weak acid function, and of (ii) 5 to 95 molar percent of at least one monomer performing a strong acid function, wherein such composition exhibits a fraction of water-soluble polymers ranging from 5 to 50 percent by weight (of the total polymer) is added to the composition prior to use.
